# EUROPEAN PATENT SPECIFICATION

(11) **EP 2 094 810 B1**
(45) Date of publication and mention of the grant of the patent: **09.08.2017**
(21) Application number: 07849981.1
(22) Date of filing: 27.11.2007
(51) Int. Cl.: C09K 11/06, H01L 51/50

(54) **ORGANIC ELECTROLUMINESCENT DEVICE AND INDOLE DERIVATIVE**
ORGANISCHE LEUCHTVORRICHTUNG SOWIE INDOLDERIVAT
DEL ORGANIQUE ET DÉRIVÉ D'INDOLE

(30) Priority: 27.11.2006 JP 2006318773; 28.08.2007 JP 2007221520
(43) Date of publication of application: 02.09.2009
(73) Proprietor: UDC Ireland Limited, Ballycoolin, Dublin 15 (IE)
(72) Inventor: IGARASHI, Tatsuya, Kanagawa (JP); YAGI, Kazunari, Kanagawa (JP)
(74) Representative: Maiwald Patentanwalts GmbH
(86) International application number: PCT/JP2007/073278
(87) International publication number: WO 2008/066195

(56) References cited:
- WO-A-2007/063754
- JP-A- 2003 277 743
- JP-A- 2004 281 296
- JP-A- 2005 082 701
- US-A1- 2006 145 145

## Description

### Technical Field

The present invention relates to a light-emitting device capable of emitting light by converting electric energy into light, in particular, the invention relates to an organic electroluminescent device (a light-emitting device, or an EL device). The invention further relates to an indole derivative useful to a light-emitting device.

### Background Art

Organic electroluminescent (EL) devices are attracting public attention as promising display devices for capable of emitting light of high luminance with low voltage. An important characteristic of organic electroluminescent devices is consumed electric power. Consumed electric power is represented by: "Consumed electric power = Voltage × electric current", so that the lower the value of voltage that is necessary to obtain desired brightness and the smaller the value of electric current, the lower is the consumed electric power of the device.

As one trial to lower the value of electric current flowing in a device, a light-emiting device utilizing luminescence from ortho-metalated iridium complex (Ir(ppy)₃: Tris-Ortho-Metalated Complex of Iridium(III) with 2-Phenylpyridine) is reported (e.g., refer to JP-A-2001-247859). The phosphorescent devices described therein are greatly improved in external quantum efficiency as compared with existing singlet luminescent devices, and have succeeded in making the value of electric current smaller. However, they cannot be said to have sufficient performances with respect to durability and efficiency, and color tone worsens with the deterioration of the device, so that further improvement is required.

For the purpose of improving the efficiency and durability of a phosphorescent device, a device containing an indole derivative (JP-A-2002-305084) is reported. However, in view of durability and efficiency, a further improvement is required.

### Disclosure of the Invention

An object of the invention is to provide a light-emitting device showing good durability and efficiency, and little in variation of chromaticity by aging. A further object is to provide a novel indole derivative.

The above objects can be achieved by the following means.
(1) An organic electroluminescent device comprising:
   a pair of electrodes; and
   at least one organic layer between the pair of electrodes, the at least one organic layer including a light-emitting layer containing a light-emitting material,
   wherein the at least one organic layer includes at least one layer containing an indole derivative represented by formula (1): 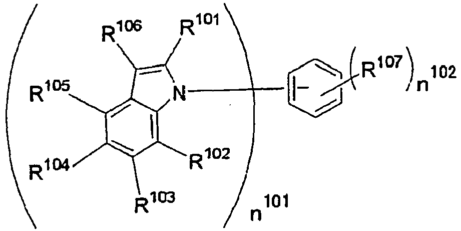 wherein R¹⁰², R¹⁰³, R¹⁰⁴, R¹⁰⁵ and R¹⁰⁶ each independently represents a hydrogen atom or a substituent; R¹⁰¹ represents a substituent linking via a carbon atom; R¹⁰¹ and R¹⁰⁶ may be bonded to each other to form a ring; R¹⁰⁷ represents a substituent; R¹⁰¹ represents 2; and n¹⁰² represents an integer of from 0 to 5, provided that n¹⁰¹ + n¹⁰² ≦ 6.
(2) The organic electroluminescent device according to (1), wherein R¹⁰¹ is an alkyl group having a tertiary or quaternary carbon atom.
(3) The organic electroluminescent device according to (1), wherein R¹⁰¹ represents a t-alkyl group.
(4) The organic electroluminescent device according to any one of (1) to (3), wherein the indole derivative is a compound represented by formula (2): 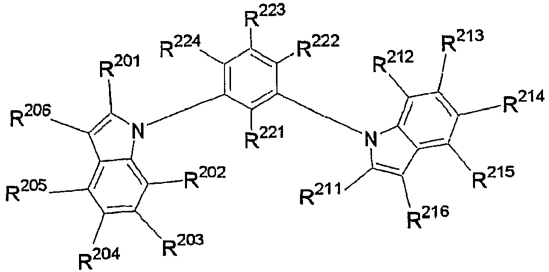 wherein R²⁰², R²⁰³, R²⁰⁴, R²⁰⁵ and R²⁰⁶ each indepednetly represents a hydrogen atom or a substituent; R²⁰¹ represents a substituent linking via a carbon atom; R²⁰¹ and R²⁰⁶ may be bonded to each other to form a ring; R²¹², R²¹³, R²¹⁴, R²¹⁵ and R²¹⁶ each independently represents a hydrogen atom or a substituent; R²¹¹ represents a substituent linking via a carbon atom; R²¹¹ and R²¹⁶ may be bonded to each other to form a ring; and R²²¹, R²²², R²²³ and R²²⁴ each independently represents a hydrogen atom or a substituent linking via a carbon atom.
(5) The organic electroluminescent device according to any one of (1) to (3), wherein the indole derivative is a compound represented by formula (3): 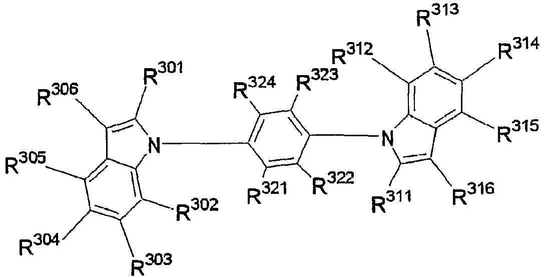 wherein R³⁰², R³⁰³, R³⁰⁴, R³⁰⁵ and R³⁰⁶ each independently represents a hydrogen atom or a substituent; R³⁰¹ represents a substituent linking via a carbon atom; R³⁰¹ and R³⁰⁶ may be bonded to each other to form a ring; R³¹², R³¹³, R³¹⁴, R³¹⁵ and R³¹⁶ each independently represents a hydrogen atom or a substituent; R³¹¹ represents a substituent linking via a carbon atom; R³¹¹ and R³¹⁶ may be bonded to each other to form a ring; and R³²¹, R³²², R³²³ and R³²⁴ each independently represents a hydrogen atom or a substituent linking via a carbon atom.
(6) The organic electroluminescent device according to any one of (1) to (3), wherein the indole derivative is a compound represented by formula (4): 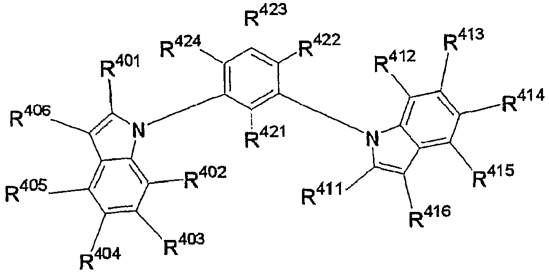 wherein R⁴⁰², R⁴⁰³, R⁴⁰⁴, R⁴⁰⁵ and R⁴⁰⁶ each independently represents a hydrogen atom or a substituent; R⁴⁰¹ represents a t-alkyl group; R⁴⁰¹ and R⁴⁰⁶ may be bonded to each other to form a ring; R⁴¹², R⁴¹³, R⁴¹⁴ R⁴¹⁵ and R⁴¹⁶ each independently represents a hydrogen atom or a substituent; R⁴¹¹ represents a t-alkyl group; R⁴¹¹ and R⁴¹⁶ may be bonded to each other to form a ring; and R⁴²¹, R⁴²², R⁴²³ and R⁴²⁴ each independently represents a hydrogen atom or a substituent linking via a carbon atom.
(7) The organic electroluminescent device according to any one of (1) to (3), wherein the indole derivative is a compound represented by formula (5): 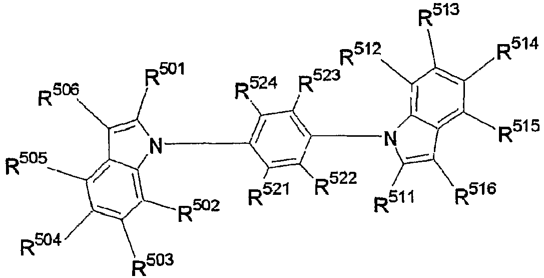 wherein R⁵⁰², R⁵⁰³, R⁵⁰⁴, R⁵⁰⁵ and R⁵⁰⁶ each independently represents a hydrogen atom or a substituent; R⁵⁰¹ represents a t-alkyl group; R⁵⁰¹ and R⁵⁰⁶ may be bonded to each other to form a ring; R⁵¹², R⁵¹³, R⁵¹⁴, R⁵¹⁵ and R⁵¹⁶ each independently represents a hydrogen atom or a substituent; R⁵¹¹ represents a t-alkyl group; R⁵¹¹ and R⁵¹⁶ may be bonded to each other to form a ring; and R⁵²¹, R⁵²², R⁵²³ and R⁵²⁴ independently each represents a hydrogen atom or a substituent linking via a carbon atom.
(8) The organic electroluminescent according to any one of (1) to (7), wherein the indole derivative is contained in the light-emitting layer.
(9) The organic electroluminescent device according to any one of (1) to (8), wherein the indole derivative is contained in a layer contiguous to the light-emitting layer.
(10) A compound represented by formula (4): 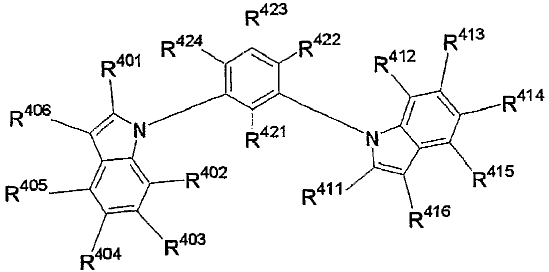 wherein R⁴⁰², R⁴⁰³, R⁴⁰⁴, R⁴⁰⁵ and R⁴⁰⁶ each independently represents a hydrogen atom or a substituent; R⁴⁰¹ represents a t-alkyl group; R⁴⁰¹ and R⁴⁰⁶ may be bonded to each other to form a ring; R⁴¹², R⁴¹³, R⁴¹⁴, R⁴¹⁵ and R⁴¹⁶ each independently represents a hydrogen atom or a substituent; R⁴¹¹ represents a t-alkyl group; R⁴¹¹ and R⁴¹⁶ may be bonded to each other to form a ring; and R⁴²¹, R⁴²², R⁴²³ and R⁴²⁴ each independently represents a hydrogen atom or a substituent linking via a carbon atom.
(11) A compound represented by formula (5): 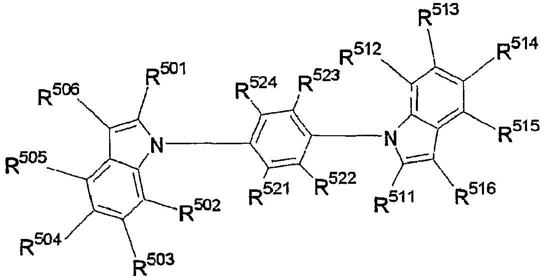 wherein R⁵⁰², R⁵⁰³, R⁵⁰⁴, R⁵⁰⁵ and R⁵⁰⁶ each independently represents a hydrogen atom or a substituent; R⁵⁰¹ represents a t-alkyl group; R⁵⁰¹ and R⁵⁰⁶ may be bonded to each other to form a ring; R⁵¹², R⁵¹³, R⁵¹⁴, R⁵¹⁵ and R⁵¹⁶ each independently represents a hydrogen atom or a substituent; R⁵¹¹ represents a t-alkyl group; R⁵¹¹ and R⁵¹⁶ may be bonded to each other to form a ring; and R⁵²¹, R⁵²², R⁵²³ and R⁵²⁴ independently each represents a hydrogen atom or a substituent linking via a carbon atom.

A light-emitting device according to an aspect of the invention is capable of light emission of high efficiency, excellent in durability, and little in hue variation by aging. Further, a novel indole derivative according to an aspect of the invention is useful as a material of the light-emitting device.

### Best Mode for Carrying Out the Invention

An aspect of the invention relates to an organic electroluminescent device including: a pair of electrodes; and at least one organic layer including a light-emitting layer, between the pair of electrodes. The at least one organic layer includes at least one layer containing an indole derivative represented by formula (1).

The compound represented by formula (1) will be explained below.

R¹⁰² to R¹⁰⁶ each independently represents a hydrogen atom or a substituent.

The examples of the substituents include an alkyl group (preferably having from 1 to 30 carbon atoms, more preferably from 1 to 20 carbon atoms, and especially preferably from 1 to 10 carbon atoms, e.g., methyl, ethyl, isopropyl, n-octyl, n-decyl, n-hexadecyl, cyclopropyl, cyclopentyl, cyclohexyl, etc., are exemplified), an alkenyl group (preferably having from 2 to 30 carbon atoms, more preferably from 2 to 20 carbon atoms, and especially preferably from 2 to 10 carbon atoms, e.g., vinyl, allyl, 2-butenyl, 3-pentenyl, etc., are exemplified), an alkynyl group (preferably having from 2 to 30 carbon atoms, more preferably from 2 to 20 carbon atoms, and especially preferably from 2 to 10 carbon atoms, e.g., propargyl, 3-pentynyl, etc., are exemplified), an aryl group (preferably having from 6 to 30 carbon atoms, more preferably from 6 to 20 carbon atoms, and especially preferably from 6 to 12 carbon atoms, e.g., phenyl, p-methylphenyl, naphthyl, anthranyl, etc., are exemplified), an amino group (preferably having from 0 to 30 carbon atoms, more preferably from 0 to 20 carbon atoms, and especially preferably from 0 to 10 carbon atoms, e.g., amino, methylamino, dimethylamino, diethylamino, dibenzylamino, diphenylamino, ditolylamino, etc., are exemplified), an alkoxyl group (preferably having from 1 to 30 carbon atoms, more preferably from 1 to 20 carbon atoms, and especially preferably from 1 to 10 carbon atoms, e.g., methoxy, ethoxy, butoxy, 2-ethylhexyloxy, etc., are exemplified), an aryloxy group (preferably having from 6 to 30 carbon atoms, more preferably from 6 to 20 carbon atoms, and especially preferably from 6 to 12 carbon atoms, e.g., phenyloxy, 1-naphthyloxy, 2-naphthyloxy, etc., are exemplified), a heterocyclic oxy group (preferably having from 1 to 30 carbon atoms, more preferably from 1 to 20 carbon atoms, and especially preferably from 1 to 12 carbon atoms, e.g., pyridyloxy, pyrazyloxy, pyrimidyloxy, quinolyloxy, etc., are exemplified), an acyl group (preferably having from 1 to 30 carbon atoms, more preferably from 1 to 20 carbon atoms, and especially preferably from 1 to 12 carbon atoms, e.g., acetyl, benzoyl, formyl, pivaloyl, etc., are exemplified), an alkoxycarbonyl group (preferably having from 2 to 30 carbon atoms, more preferably from 2 to 20 carbon atoms, and especially preferably from 2 to 12 carbon atoms, e.g., methoxycarbonyl, ethoxycarbonyl, etc., are exemplified), an aryloxycarbonyl group (preferably having from 7 to 30 carbon atoms, more preferably from 7 to 20 carbon atoms, and especially preferably from 7 to 12 carbon atoms, e.g., phenyloxycarbonyl, etc., are exemplified), an acyloxy group (preferably having from 2 to 30 carbon atoms, more preferably from 2 to 20 carbon atoms, and especially preferably from 2 to 10 carbon atoms, e.g., acetoxy, benzoyloxy, etc., are exemplified), an acylamino group (preferably having from 2 to 30 carbon atoms, more preferably from 2 to 20 carbon atoms, and especially preferably from 2 to 10 carbon atoms, e.g., acetylamino, benzoylamino, etc., are exemplified), an alkoxycarbonylamino group (preferably having from 2 to 30 carbon atoms, more preferably from 2 to 20 carbon atoms, and especially preferably from 2 to 12 carbon atoms, e.g., methoxycarbonylamino, etc., are exemplified), an aryloxycarbonylamino group (preferably having from 7 to 30 carbon atoms, more preferably from 7 to 20 carbon atoms, and especially preferably from 7 to 12 carbon atoms, e.g., phenyloxycarbonylamino, etc., are exemplified), a sulfonylamino group (preferably having from 1 to 30 carbon atoms, more preferably from 1 to 20 carbon atoms, and especially preferably from 1 to 12 carbon atoms, e.g., methanesulfonylamino, benzenesulfonylamino, etc., are exemplified), a sulfamoyl group (preferably having from 0 to 30 carbon atoms, more preferably from 0 to 20 carbon atoms, and especially preferably from 0 to 12 carbon atoms, e.g., sulfamoyl, methylsulfamoyl, dimethylsulfamoyl, phenylsulfamoyl, etc., are exemplified), a carbamoyl group (preferably having from 1 to 30 carbon atoms, more preferably from 1 to 20 carbon atoms, and especially preferably from 1 to 12 carbon atoms, e.g., carbamoyl, methylcarbamoyl, diethylcarbamoyl, phenylcarbamoyl, etc., are exemplified), an alkylthio group (preferably having from 1 to 30 carbon atoms, more preferably from 1 to 20 carbon atoms, and especially preferably from 1 to 12 carbon atoms, e.g., methylthio, ethylthio, etc., are exemplified), an arylthio group (preferably having from 6 to 30 carbon atoms, more preferably from 6 to 20 carbon atoms, and especially preferably from 6 to 12 carbon atoms, e.g., phenylthio, etc., are exemplified), a heterocyclic thio group (preferably having from 1 to 30 carbon atoms, more preferably from 1 to 20 carbon atoms, and especially preferably from 1 to 12 carbon atoms, e.g., pyridylthio, 2-benzimizolylthio, 2-benzoxazolylthio, 2-benzothiazolylthio, etc., are exemplified), a sulfonyl group (preferably having from 1 to 30 carbon atoms, more preferably from 1 to 20 carbon atoms, and especially preferably from 1 to 12 carbon atoms, e.g., mesyl, tosyl, etc., are exemplified), a sulfinyl group (preferably having from 1 to 30 carbon atoms, more preferably from 1 to 20 carbon atoms, and especially preferably from 1 to 12 carbon atoms, e.g., methanesulfinyl, benzenesulfinyl, etc., are exemplified), a ureido group (preferably having from 1 to 30 carbon atoms, more preferably from 1 to 20 carbon atoms, and especially preferably from 1 to 12 carbon atoms, e.g., ureido, methylureido, phenylureido, etc., are exemplified), a phosphoric amido group (preferably having from 1 to 30 carbon atoms, more preferably from 1 to 20 carbon atoms, and especially preferably from 1 to 12 carbon atoms, e.g., diethylphosphoric amido, phenylphosphoric amido, etc., are exemplified), a hydroxyl group, a mercapto group, a halogen atom (e.g., a fluorine atom, a chlorine atom, a bromine atom, an iodine atom), a cyano group, a sulfo group, a carboxyl group, a nitro group, a hydroxamic acid group, a sulfino group, a hydrazino group, an imino group, a heterocyclic group (preferably having from 1 to 30 carbon atoms, and more preferably from 1 to 20 carbon atoms, and as the hetero atoms, e.g., a nitrogen atom, an oxygen atom, a sulfur atom are exemplified, specifically, e.g., imidazolyl, pyridyl quinolyl, furyl, thienyl, piperidyl, morpholino, benzoxazolyl, benzimidazolyl, benzothiazolyl, carbazolyl, azepinyl, etc., are exemplified), a silyl group (preferably having from 3 to 40 carbon atoms, more preferably from 3 to 30 carbon atoms, and especially preferably from 3 to 24 carbon atoms, e.g., trimethylsilyl, triphenylsilyl, etc., are exemplified), and a silyloxy group (preferably having from 3 to 40 carbon atoms, more preferably from 3 to 30 carbon atoms, and especially preferably from 3 to 24 carbon atoms, e.g., trimethylsilyloxy, triphenylsilyloxy, etc., are exemplified). These substituents may further be substituted.

R¹⁰² to R¹⁰⁶ each preferably represents a hydrogen atom, an alkyl group, an aryl group, or a hetero aryl group, more preferably a hydrogen atom, an alkyl group, or an aryl group, still more preferably a hydrogen atom or an alkyl group, and especially preferably a hydrogen atom.

R¹⁰¹ represents a substituent linking via a carbon atom (i.e., a substituent linking to an indole ring via a carbon atom) (the number of carbon atoms of R¹⁰¹ is preferably from 1 to 15, more preferably from 3 to 10, and still more preferably from 4 to 6). R¹⁰¹ preferably represents an alkyl group, an aryl group, or a hetero aryl group linking via a carbon atom, more preferably an alkyl group or an aryl group, and still more preferably an alkyl group. As the alkyl group, preferably an alkyl group having a tertiary or quaternary carbon atom (e.g., a t-butyl group, an isopropyl group, an isobutyl group, an isopentyl group, a cyclopropyl group, a cyclopentyl group, a cyclohexyl group, a 2-phenyl-2-propyl group, a 2-(2-pyridyl)propyl group, etc.), more preferably an alkyl group having a quaternary carbon atom, and especially preferably a t-alkyl group. As the t-alkyl group, a t-butyl group is preferred. Further, R¹⁰¹ and R¹⁰⁶ may be bonded to each other to form a ring. As the ring formed by R¹⁰¹ and R¹⁰⁶ to be bonded to each other, e.g., a cyclopentene ring, a cyclohexene ring, a 1,4-cyclohexadiene ring, a cycloheptene ring, a cyclooctene ring, etc., are exemplified.

R¹⁰¹ may have a substituent. As the substituents, those exemplified as the substituents represented by R¹⁰² to R¹⁰⁶ are applicable.

R¹⁰⁷ represents a substituent. As the substituent, the groups described in R¹⁰² are exemplified, and an alkyl group, an aryl group and a silyl group are preferred, an alkyl group and a silyl group having a aryl group as a substituent are more preferred, and an alkyl group is still more preferred.

n¹⁰¹ represents 2. When n¹⁰² is 2, a plurality of indole skeletal parts may be the same or different. n¹⁰² represents an integer of from 0 to 5, preferably 0 to 3, more preferably 0 or 1, and still more preferably 0, provided that n¹⁰¹ + n¹⁰² ≦ 6.

The compound represented by formula (1) is preferably a compound represented by formula (2) or (3), and more preferably a compound represented by formula (2).

The compound represented by formula (2) will be described below.

R²⁰¹ to R²⁰⁶ each has the same meaning as the meaning of R¹⁰¹ to R¹⁰⁶, and the preferred range is also the same. R²⁰¹ and R²⁰⁶ may be bonded to each other to form a ring. As the ring formed by R²⁰¹ and R²⁰⁶ to be bonded to each other, e.g., a cyclopentene ring, a cyclohexene ring, a 1,4-cyclohexadiene ring, a cyclopeptene ring, a cyclooctene ring, etc., are exemplified.

R²⁰¹ representing a substituent linking via a carbon atom may have a further substituent. As the substituents, those exemplified as the substituents represented by R¹⁰² to R¹⁰⁶ are applicable.

R²¹¹ to R²¹⁶ each has the same meaning as the meaning of R¹⁰¹ to R¹⁰⁶, and the preferred range is also the same. R²¹¹ and R²¹⁶ may be bonded to each other to form a ring. As the ring formed by R²¹¹ and R²¹⁶ to be bonded to each other, e.g., a cyclopentene ring, a cyclohexene ring, a 1,4-cyclohexadiene ring, a cyclopeptene ring, a cyclooctene ring, etc., are exemplified.

R²¹¹ representing a substituent linking via a carbon atom may have a further substituent. As the substituents, those exemplified as the substituents represented by R¹⁰² to R¹⁰⁶ are applicable.

R²²¹ to R²²⁴ each represents a hydrogen atom or a substituent linking via a carbon atom (preferably having from 1 to 15 carbon atoms, more preferably from 1 to 10, and still more preferably from 1 to 4). R²²¹ to R²²⁴ each preferably represents a hydrogen atom, an alkyl group, or an aryl group, more preferably a hydrogen atom or an alkyl group, and still more preferably a hydrogen atom.

The compound represented by formula (2) is preferably a compound represented by formula (4).

Formula (3) is described below.

R³⁰¹ to R³⁰⁶ each has the same meaning as the meaning of R¹⁰¹ to R¹⁰⁶, and the preferred range is also the same. Further, R³⁰¹ and R³⁰⁶ may be bonded to each other to form a ring. As the ring formed by R³⁰¹ and R³⁰⁶ to be bonded to each other, e.g., a cyclopentene ring, a cyclohexene ring, a 1,4-cyclohexadiene ring, a cyclopeptene ring, a cyclooctene ring, etc., are exemplified.

R³⁰¹ representing a substituent linking via a carbon atom may have a further substituent. As the substituents, those exemplified as the substituents represented by R¹⁰² to R¹⁰⁶ are applicable.

R³¹¹ to R³¹⁶ each has the same meaning as that of R¹⁰¹ to R¹⁰⁶, and the preferred range is also the same. Further, R³¹¹ and R³¹⁶ may be bonded to each other to form a ring. As the ring formed by R³¹¹ and R³¹⁶ to be bonded to each other, e.g., a cyclopentene ring, a cyclohexene ring, a 1,4-cyclohexadiene ring, a cyclopeptene ring, a cyclooctene ring, etc., are exemplified.

R³¹¹ representing a substituent linking via a carbon atom may have a further substituent. As the substituents, those exemplified as the substituents represented by R¹⁰² to R¹⁰⁶ are applicable.

R³²¹ to R³²⁴ each represents a hydrogen atom or a substituent linking via carbon atom (having preferably 1 to 15 carbon atoms, more preferably 1 to 10 carbon atoms, and still more preferably 1 to 4 carbon atoms). As R³²¹ to R³²⁴, a hydrogen atom, a alkyl group and a aryl group are preferred, and a hydrogen atom and a alkyl group are more preferred.

Formula (4) is described below.

R⁴⁰² to R⁴⁰⁶ each has the same meaning as the meaning of R¹⁰² to R¹⁰⁶, and the preferred range is also the same.

R⁴¹² to R⁴¹⁶ each has the same meaning as that of R¹⁰² to R¹⁰⁶, and the preferred range is also the same.

R⁴²¹ to R⁴²⁴ each has the same meaning as that of R²²¹ to R²²⁴, and the preferred range is also the same.

R⁴⁰¹ and R⁴¹¹ each represents a t-alkyl group, and preferably a t-butyl group and 2-methyl-2-butyl group, and more preferably a t-butyl group. Further, R⁴⁰¹ and R⁴⁰⁶ may be bonded to each other to form a ring, and R⁴¹¹ and R⁴¹⁶ may be bonded to each other to form a ring. As the ring formed by R⁴⁰¹ and R⁴⁰⁶ or R⁴¹¹ and R⁴¹⁶ to be bonded to each other, e.g., a cyclopentene ring, a cyclohexene ring, a 1,4-cyclohexadiene ring, a cyclopeptene ring, a cyclooctene ring, etc., are exemplified.

Formula (5) is described below.

R⁵⁰² to R⁵⁰⁶ each has the same meaning as the meaning of R¹⁰² to R¹⁰⁶, and the preferred range is also the same.

R⁵¹² to R⁵¹⁶ each has the same meaning as that of R¹⁰² to R¹⁰⁶, and the preferred range is also the same.

R⁵²¹ to R⁵²⁴ each has the same meaning as that of R²²¹ to R²²⁴, and the preferred range is also the same.

R⁵⁰¹ and R⁵¹¹ each represents a t-alkyl group, and preferably a t-butyl group and 2-methyl-2-butyl group, and more preferably a t-butyl group. Further, R⁵⁰¹ and R⁵⁰⁶ may be bonded to each other to form a ring, and R⁵¹¹ and R⁵¹⁶ may be bonded to each other to form a ring. As the ring formed by R⁵⁰¹ and R⁵⁰⁶ or R⁵¹¹ and R⁵¹⁶ to be bonded to each other, e.g., a cyclopentene ring, a cyclohexene ring, a 1,4-cyclohexadiene ring, a cyclopeptene ring, a cyclooctene ring, etc., are exemplified.

The indole derivative of the invention preferably has one or two indole skeletons, and more preferably has two indole skeletons.

The indole derivative represented by any of formulae (1) to (5) is preferably contained in the light-emitting layer or a layer contiguous to the light-emitting layer, and more preferably contained in the light-emitting layer or a layer contiguous to the light-emitting layer on the anode side. It is also preferred to introduce the indole derivative represented by any of formulae (1) to (5) into both of the light-emitting layer and a layer contiguous to the light-emitting layer on the anode side.

When the indole derivative represented by any of formulae (1) to (5) is contained in the light-emitting layer, the content is preferably from 50 to 99 mass% (weight%), more preferably from 60 to 95 mass%, and still more preferably from 70 to 90 mass%. When the indole derivative is contained in layers other than the light-emitting layer, the content is preferably from 20 to 100 mass%, more preferably from 60 to 100 mass%, and still more preferably from 90 to 100 mass%.

By the introduction of the indole derivative represented by any of formulae (1) to (5) into the light-emitting device, it becomes possible to obtain good efficiency and durability and prevent variation of chromaticity by aging.

The indole derivatives for use in the invention may be low molecular weight compounds, may be high molecular weight compounds in which the residue is directly bonded to the polymer main chain (preferably having a mass average molecular weight of from 1,000 to 5,000,000, more preferably from 5,000 to 2,000,000, and still more preferably from 10,000 to 1,000,000), or may be high molecular weight compounds having the indole derivative of the invention on the main chain (preferably having a mass average molecular weight of from 1,000 to 5,000,000, more preferably from 5,000 to 2,000,000, and still more preferably from 10,000 to 1,000,000). In the case where the indole derivatives are high molecular weight compounds, they may be homopolymers, or may be copolymers with other polymers. When the indole derivatives are copolymers, they may be random copolymers or block copolymers. Further, when they are copolymers, at least one of a compound having a luminescent function and a compound having a charge transporting function may be contained in the polymers.

It is preferred for the light-emitting device in the invention to contain a phosphorescent material, e.g., a platinum phosphorescent material, and it is more preferred to contain a platinum complex having a tetradentate ligand.

The transition metal atom is not particularly limited, but preferred examples thereof include ruthenium, rhodium, palladium, tungsten, rhenium, osmium, iridium and platinum. Among these, rhenium, iridium and platinum are more preferred. In particular, the light-emitting element preferably contains a platinum-based phosphorescent material, more preferably a tetradentate platinum complex.

Examples of the lanthanoid atom include lanthanum, cerium, praseodymium, neodymium, samarium, europium, gadolinium, terbium, dysprosium, holmium, erbium, thulium, ytterbium and lutecium. Among these lanthanoid atoms, neodymium, europium and gadolinium are preferred.

The maximum emission wavelength of the platinum complex phosphorescent material having a tetradentate ligand is preferably 500 nm or less, more preferably 480 nm or less, still more preferably 470 nm or less, and especially preferably 460 nm or less.

The external quantum efficiency of the light-emitting device is preferably 5% or more, more preferably 10% or more, and still more preferably 13% or more. As the numerical value of external quantum efficiency, the maximum value of the external quantum efficiency at the time of driving a device at 20 °C, or the value of the external quantum efficiency near 100 to 300 cd/m² at the time of driving a device at 20 °C can be used.

The inner quantum efficiency of the light-emitting device is preferably 30% or more, more preferably 50% or more, and still more preferably 70% or more. The inner quantum efficiency of a device is computed by the expression: inner quantum efficiency = external quantum efficiency/coupling out efficiency of light. In ordinary organic EL device, coupling out efficiency of light is about 20%, but it is possible to make coupling out efficiency of light 20% or more by various contrivances such as the shape of a substrate, the shape of electrodes, the thickness of an organic layer, the thickness of an inorganic layer, the refractive index of an organic layer, and the refractive index of an inorganic layer.

The light-emitting device is preferably a device having at least three layers of a hole transporting layer, a light-emitting layer and an electron transporting layer. It is more preferred to additionally provide, between the hole transporting layer and the light-emitting layer, a layer to accelerate hole injection to the light-emitting layer, a layer to block electrons, and a layer to block excitons.

The degree of charge transfer of a host material contained in the light-emitting layer is preferably 1×10⁻⁶ cm²/Vs or more and 1×10⁻¹ cm²/Vs or less, more preferably 5×10⁻⁶ cm²/Vs or more and 1×10⁻² cm²/Vs or less, still more preferably 1×10⁻⁵ cm²/Vs or more and 1×10⁻² cm²/Vs or less, and especially preferably 5×10⁻⁵ cm²/Vs or more and 1×10⁻² cm²/Vs or less.

The glass transition points of host materials, electron transporting materials and hole transporting materials contained in the organic electroluminescent device are preferably 90 °C or more and 400 °C or less, more preferably 100 °C or more and 380 °C or less, still more preferably 120 °C or more and 370 °C or less, and especially preferably 140 °C or more and 360 °C or less.

The organic electroluminescent device of the invention may be a white luminescent device.

The T₁ level (the energy level in the state of minimum triplet excitation) of the host material contained in the light-emitting device of the invention is preferably 60 kcal/mol or more (251.4 kJ/mol or more) and 90 kcal/mol or less (377.1 kJ/mol or less), more preferably 62 kcal/mol or more (259.78 kJ/mol or more) and 85 kcal/mol or less (356.15 kJ/mol or less), and still more preferably 65 kcal/mol or more (272.35 kJ/mol or more) and 80 kcal/mol or less (335.2 kJ/mol or less).

T₁ energy can be found by measuring the spectrum of emission of phosphorescence of a thin film of the material, and from the end of the short wavelength. For example, a film is formed with the material on a cleaned quartz glass substrate in a thickness of about 50 nm by vacuum deposition. The spectrum of emission of phosphorescence of the thin film is measured with a fluorescence spectrophotometer Model F-7000 (manufactured by Hitachi High Technologies) under a liquid nitrogen temperature. The T₁ energy can be found by converting the rising wavelength on the short wave side of the obtained emission spectrum into an energy unit.

The T₁ level (the energy level in the state of minimum triplet excitation) of the layer contiguous to the light-emitting layer is preferably 60 kcal/mol or more (251.4 kJ/mol or more) and 90 kcal/mol or less (377.1 kJ/mol or less), more preferably 62 kcal/mol or more (259.78 kJ/mol or more) and 85 kcal/mol or less (356.15 kJ/mol or less), and still more preferably 65 kcal/mol or more (272.35 kJ/mol or more) and 80 kcal/mol or less (335.2 kJ/mol or less).

The compounds according to the invention are shown below, but the invention is not restricted to these compounds.

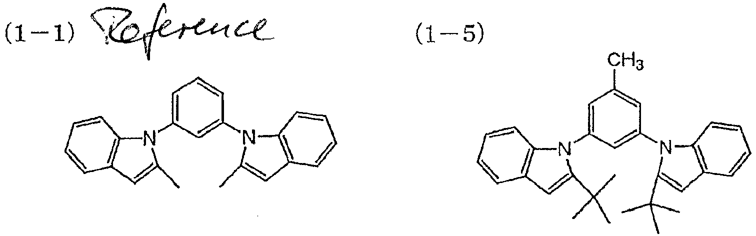

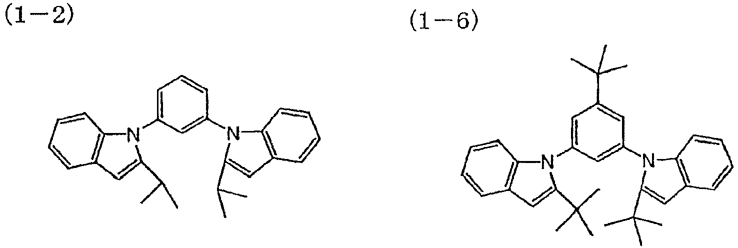

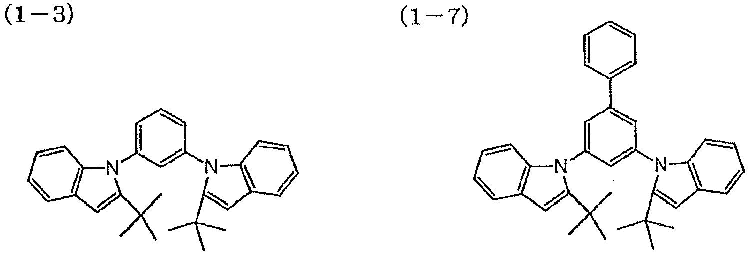

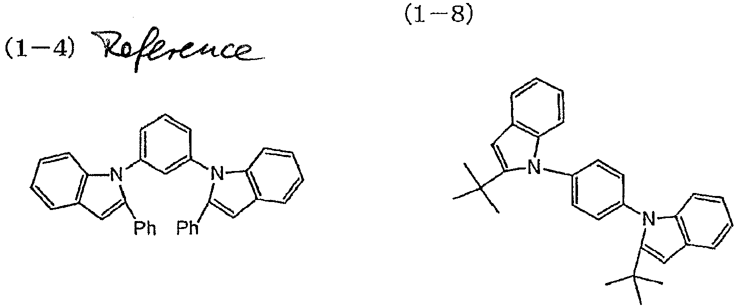

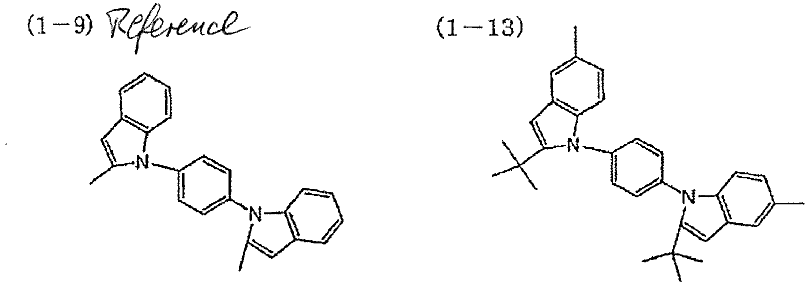

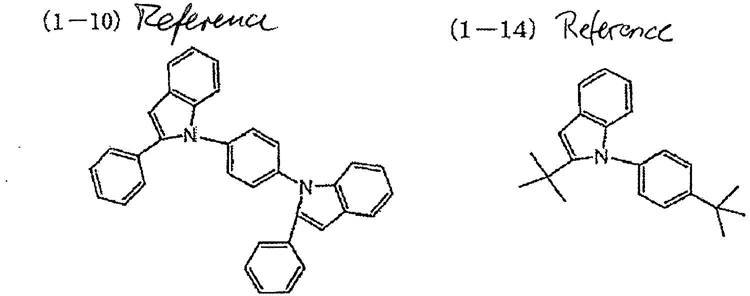

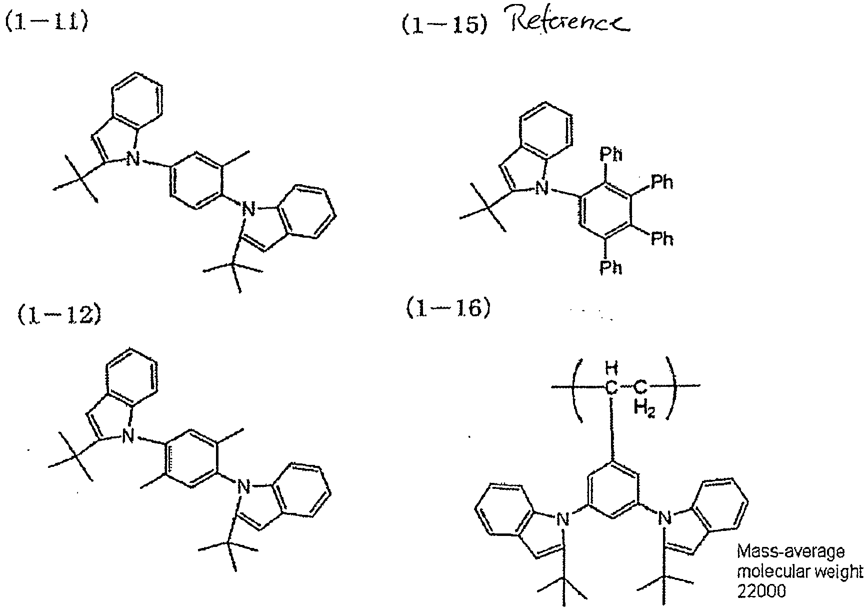

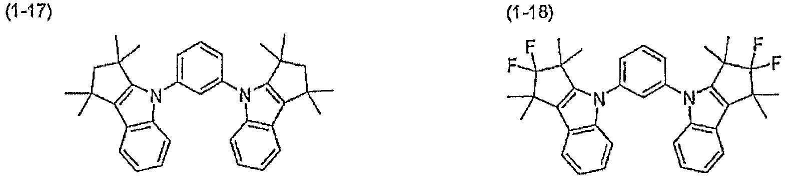

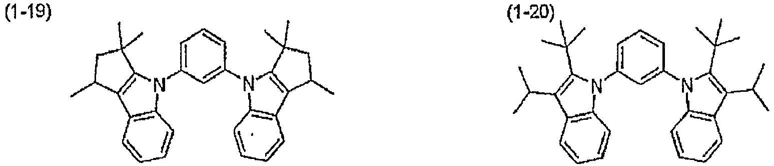

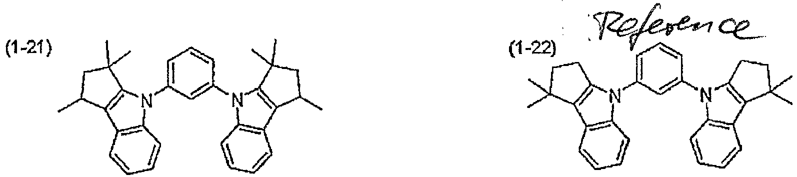

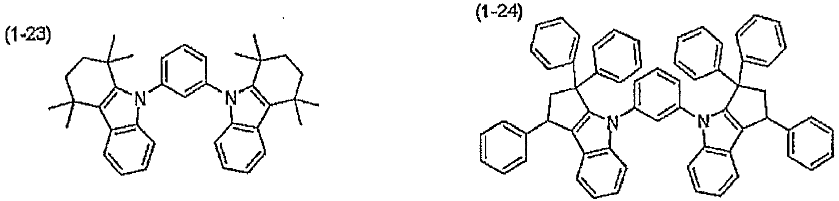

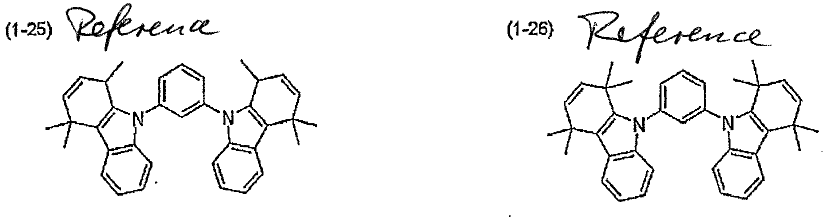

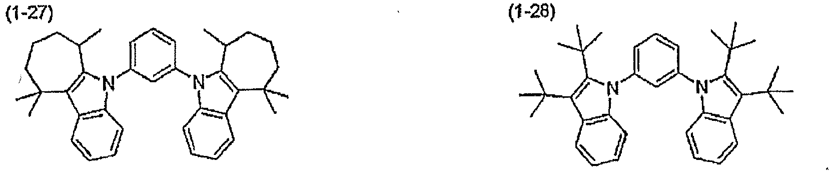

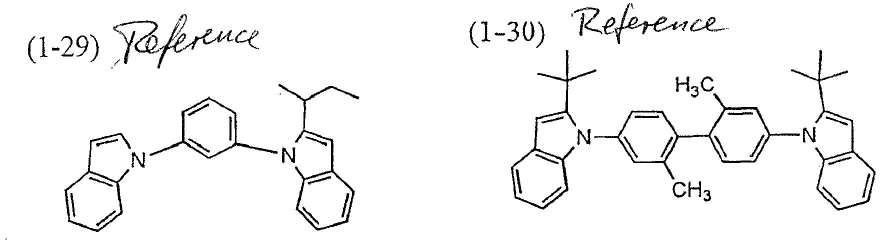

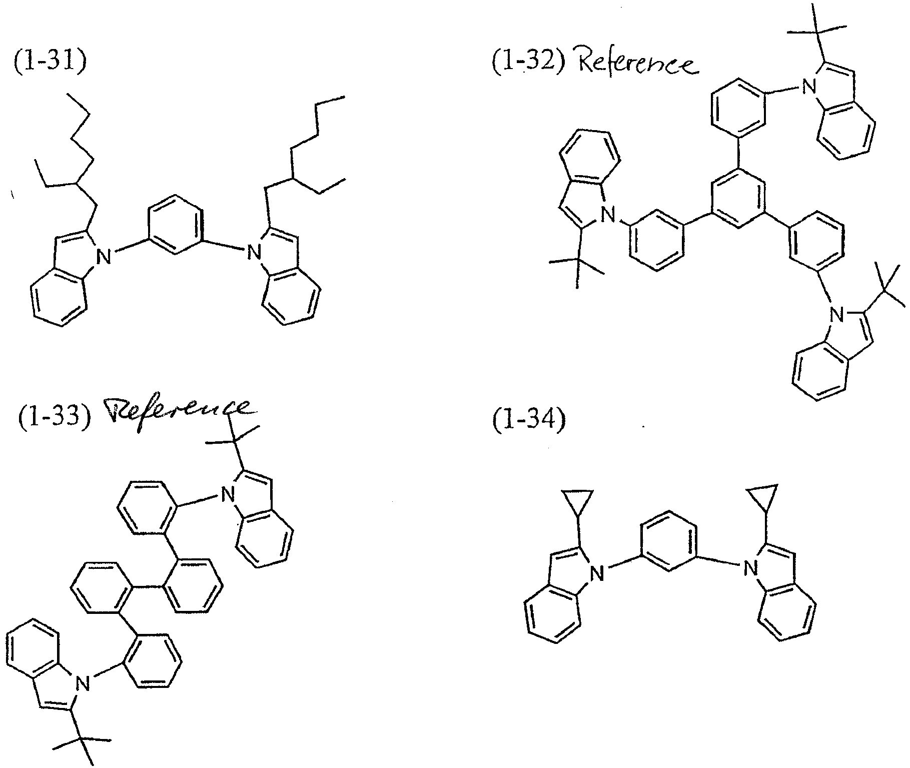

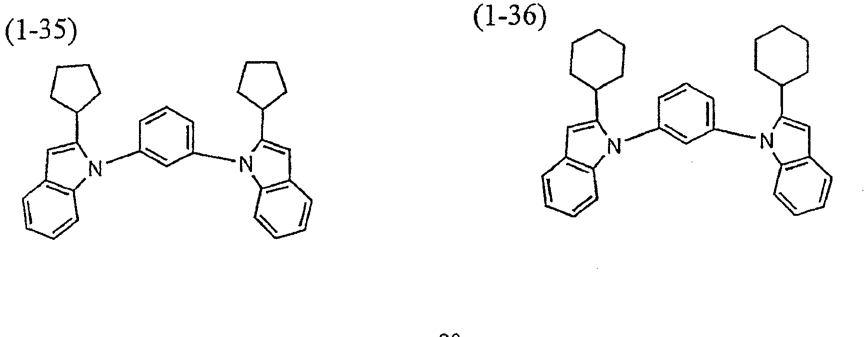

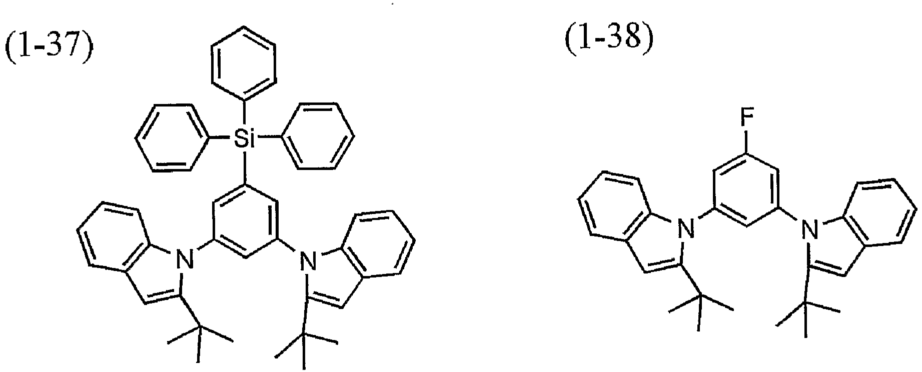

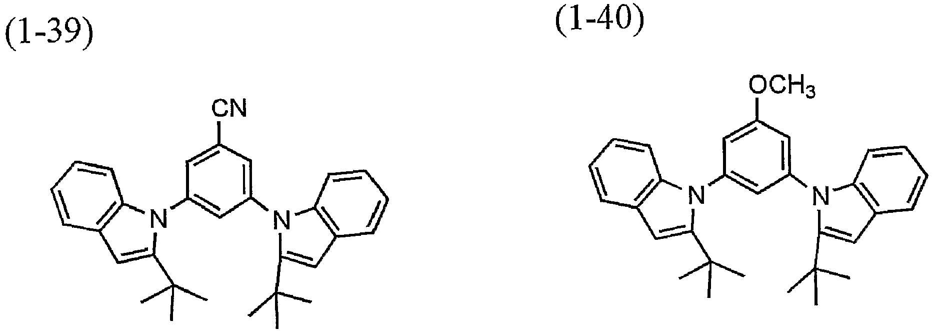

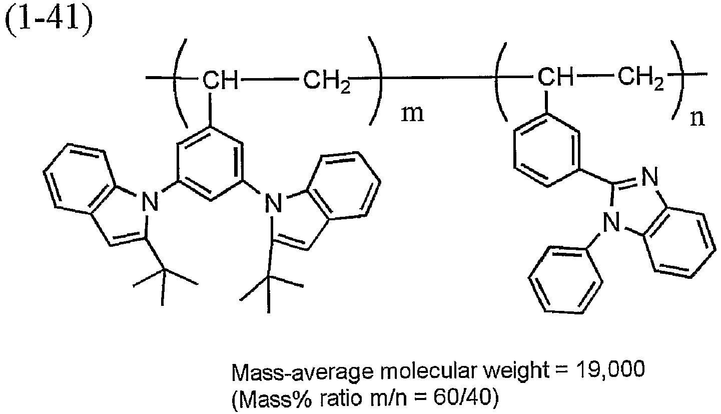

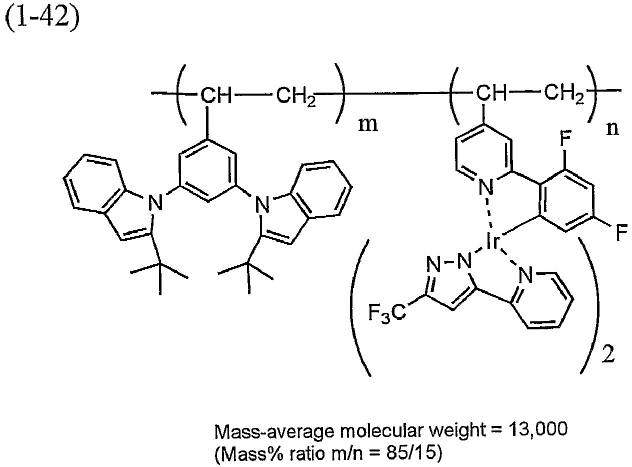

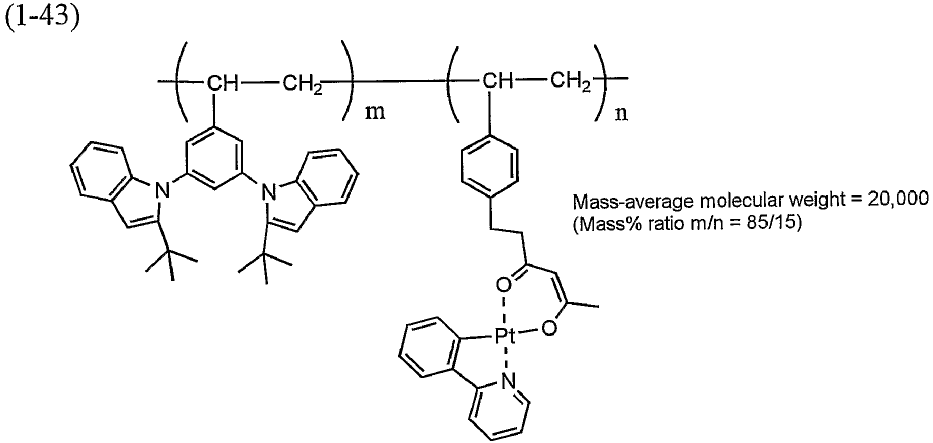

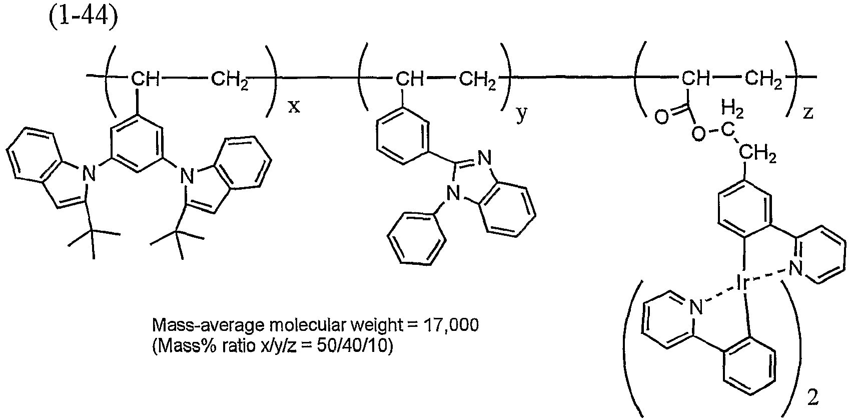

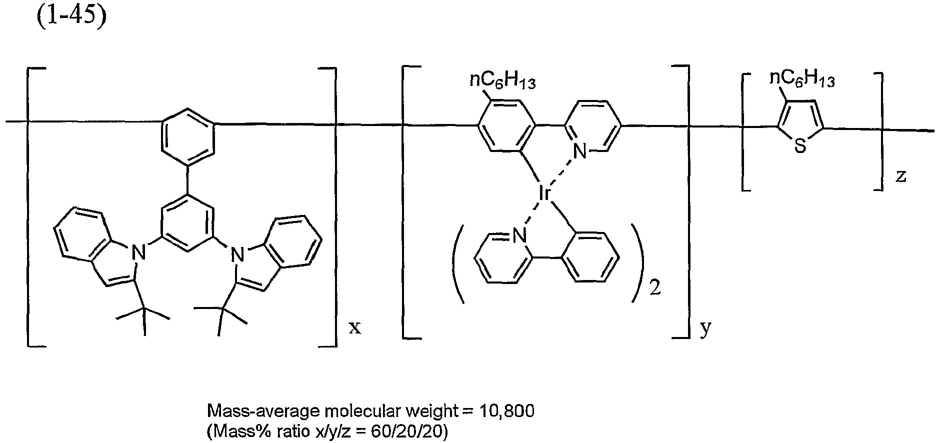

The compounds in the invention can be synthesized by various synthesizing methods. The synthesizing methods are not especially limited, and the compounds can be synthesized by referring, for example, to Org. Lett., 2005, 3, 439.

### Organic electroluminescent device:

The organic electroluminescent devices in the invention are described in detail below.

The light-emitting device includes a substrate having thereon a cathode and an anode, and organic layers (the organic layers may be organic layers including an organic compound alone, or may be organic layers containing an inorganic compound) including an organic light-emitting layer (sometimes referred to as merely "a light-emitting layer") between the electrodes. From the properties of the light-emitting device, it is preferred that at least one electrode of the cathode and anode is transparent.

As the embodiment of stacking of the organic layers in the invention, stacking is preferably in order of a hole transporting layer, a light-emitting layer, and an electron transporting layer from the anode side. Further, a charge blocking layer may be provided between the hole transporting layer and the light-emitting layer, or between the light-emitting layer and the electron transporting layer. A hole injecting layer may be provided between the anode and the hole transporting layer, and an electron injecting layer may be provided between the cathode and the electron transporting layer. Each layer may be divided into a plurality of secondary layers.

Constituents of the light-emitting device in the invention are described in detail below.

### Substrate:

The substrate for use in the invention is preferably a substrate that does not scatter or attenuate the light emitted from the organic layer. The specific examples of the materials of the substrate include inorganic materials, e.g., yttria stabilized zirconia (YSZ), glass, etc., and organic materials, such as polyester, e.g., polyethylene terephthalate, polybutylene phthalate, polyethylene naphthalate, etc., polystyrene, polycarbonate, polyether sulfone, polyallylate, polyimide, polycycloolefin, norbornene resin, poly(chloro- trifluoroethylene), etc.

When glass is used as a substrate, non-alkali glass is preferably used as the material for reducing elution of ions from the glass. Further, when soda lime glass is used, it is preferred to provide a barrier coat such as silica. In the case of organic materials, materials excellent in heat resistance, dimensional stability, solvent resistance, electrical insulating properties and processability are preferably used.

The form, structure and size of a substrate are not especially restricted, and these can be arbitrarily selected in accordance with the intended use and purpose of the light-emitting device. In general, a substrate is preferably in a plate-like form. The structure of a substrate may be a single layer structure or may be a stacking structure, and may consist of a single member or may be formed of two or more members.

A substrate may be colorless and transparent, or may be colored and transparent, but from the point of not scattering or attenuating the light emitted from an organic light-emitting layer, a colorless and transparent substrate is preferably used.

A substrate can be provided with a moisture permeation preventing layer (a gas barrier layer) on the front surface or rear surface.

As the materials of the moisture permeation preventing layer (the gas barrier layer), inorganic materials such as silicon nitride and silicon oxide are preferably used. The moisture permeation preventing layer (the gas barrier layer) can be formed, for example, by a high frequency sputtering method.

When a thermoplastic substrate is used, if necessary, a hard coat layer and an undercoat layer may further be provided.

### Anode:

An anode is generally sufficient to have the function of the electrode to supply positive holes to an organic layer. The form, structure and size of an anode are not especially restricted, and these can be arbitrarily selected from known materials of electrode in accordance with the intended use and purpose of the light-emitting device. As described above, an anode is generally provided as a transparent anode.

As the materials of anode, for example, metals, alloys, metal oxides, electrically conductive compounds, and mixtures of these materials are preferably exemplified. The specific examples of the materials of anode include electrically conductive metal oxides, e.g., tin oxide doped with antimony or fluorine (ATO, FTO), tin oxide, zinc oxide, indium oxide, indium tin oxide (ITO), indium zinc oxide (IZO), etc., metals, e.g., gold, silver, chromium, nickel, etc., mixtures or layered products of these metals with electrically conductive metal oxides, inorganic electrically conductive substances, e.g., copper iodide, copper sulfide, etc., organic electrically conductive materials, e.g., polyaniline, polythiophene, polypyrrole, etc., laminates of these materials with ITO, etc. Of these materials, electrically conductive metal oxides are preferred, and ITO is especially preferred in view of productivity, high conductivity, transparency and the like.

An anode can be formed on the substrate in accordance with various methods arbitrarily selected from, for example, wet methods, e.g., a printing method, a coating method, etc., physical methods, e.g., a vacuum deposition method, a sputtering method, an ion plating method, etc., and chemical methods, e.g., a CVD method, a plasma CVD method, etc., taking the suitability with the material to be used in the anode into consideration. For example, in the case of selecting ITO as the material of an anode, the anode can be formed according to a direct current or high frequency sputtering method, a vacuum deposition method, an ion plating method, etc.

In the organic electroluminescent device, the position of the anode to be formed is not especially restricted and can be formed anywhere. The position can be arbitrarily selected in accordance with the intended use and purpose of the light-emitting device, but preferably provided on the substrate. In this case, the anode may be formed on the entire surface of one side of the substrate, or may be formed at a part.

As patterning in forming an anode, patterning may be performed by chemical etching such as by photo-lithography, may be carried out by physical etching such as by laser, may be performed by vacuum deposition or sputtering on a superposed mask, or a lift-off method and a printing method may be used.

The thickness of an anode can be optionally selected in accordance with the materials of the anode, so that cannot be regulated unconditionally, but the thickness is generally from 10 nm to 50 µm or so, and is preferably from 50 nm to 20 µm.

The value of resistance of an anode is preferably 10³ Ω/□ or less, and more preferably 10² Ω/□ or less. In the case where an anode is transparent, the anode may be colorless and transparent, or colored and transparent. For the coupling out of luminescence from the transparent anode side, transmittance is preferably 60% or more, and more preferably 70% or more.

In connection with transparent anodes, description is found in Yutaka Sawada supervised, Tomei Denkyoku-Maku no Shintenkai (New Development in Transparent Electrode Films), CMC Publishing Co., Ltd. (1999), and the description therein can be referred to. In the case of using a plastic substrate low in heat resistance, a transparent anode film-formed with ITO or IZO at a low temperature of 150 °C or less is preferred.

### Cathode:

A cathode is generally sufficient to have the function of the electrode to supply electrons to an organic layer. The form, structure and size of a cathode are not especially restricted, and these can be arbitrarily selected from known materials of electrode in accordance with the intended use and purpose of the light-emitting device.

As the materials of cathode, for example, metals, alloys, metal oxides, electrically conductive compounds, and mixtures of these materials are exemplified. The specific examples of the materials of cathode include alkali metals (e.g., Li, Na, K, Cs, etc.), alkaline earth metals (e.g., Mg, Ca, etc.), gold, silver, lead, aluminum, sodium-potassium alloy, lithium- aluminum alloy, magnesium-silver alloy, indium, rare earth metals, e.g., ytterbium, etc. These materials may be used by one kind alone, but from the viewpoint of the compatibility of stability and an electron injecting property, two or more kinds of materials are preferably used in combination.

As the materials constituting a cathode, alkali metals and alkaline earth metals are preferred of these materials in the point of electron injection, and materials mainly comprising aluminum are preferred for their excellent preservation stability.

The materials mainly comprising aluminum mean aluminum alone, alloys of aluminum with 0.01 to 10 mass% of alkali metal or alkaline earth metal, or mixtures of these (e.g., lithium-aluminum alloy, magnesium-aluminum alloy, etc.).

The materials of cathode are disclosed in JP-A-2-15595 and JP-A-5-121172, and the materials described in these patents can also be used in the invention.

A cathode can be formed by known methods with no particular restriction. For example, a cathode can be formed according to wet methods, e.g., a printing method, a coating method, etc., physical methods, e.g., a vacuum deposition method, a sputtering method, an ion plating method, etc., and chemical methods, e.g., a CVD method, a plasma CVD method, etc., taking the suitability with the material constituting the cathode into consideration. For example, in the case of selecting metals as the material of a cathode, the cathode can be formed with one or two or more kinds of materials at the same time or in order by sputtering, etc.

As patterning in forming a cathode, patterning may be performed by chemical etching such as by photo-lithography, may be carried out by physical etching such as by laser, may be performed by vacuum deposition or sputtering on a superposed mask, or a lift-off method and a printing method may be used.

The position of the cathode to be formed is not especially restricted and can be formed anywhere in the invention. The cathode may be formed on the entire surface of the organic layer, or may be formed at a part.

A dielectric layer comprising fluoride or oxide of alkali metal or alkaline earth metal may be inserted between the cathode and the organic layer in a thickness of from 0.1 to 5 nm. The dielectric layer can be regarded as one kind of an electron injecting layer. The dielectric layer can be formed, for example, according to a vacuum deposition method, a sputtering method, an ion plating method, etc.

The thickness of a cathode can be optionally selected in accordance with the materials of the cathode, so that cannot be regulated unconditionally, but the thickness is generally from 10 nm to 5 µm or so, and is preferably from 50 nm to 1 µm.

A cathode may be transparent or opaque. A transparent cathode can be formed by forming a thin film of the materials of the cathode in a thickness of from 1 to 10 nm, and further laminating transparent conductive materials such as ITO and IZO.

### Organic layer:

Organic layers in the invention will be described below.

The organic electroluminescent device of the invention has at least one organic layer including a light-emitting layer. As organic layers other than the light-emitting layer, as described above, a hole transporting layer, an electron transporting layer, a charge blocking layer, a hole injecting layer and an electron injecting layer are exemplified.

### Formation of organic layers:

In the organic electroluminescent device of the invention, each layer constituting the organic layers can be preferably formed by any of dry film-forming methods such as a vacuum deposition method, a sputtering method, etc., a transfer method, and a printing method.

### Organic light-emitting layer:

The organic light-emitting layer is a layer having functions to receive, at the time of electric field application, positive holes from the anode, hole injecting layer or hole transporting layer, and electrons from the cathode, electron injecting layer or electron transporting layer, and offer the field of recombination of positive holes and electrons to emit light.

The light-emitting layer in the invention may consist of light-emitting materials alone, or may comprise a mixed layer of a host material and a light-emitting material. The light-emitting material may be a fluorescent material or may be a phosphorescent material. Dopant may be one or two or more kinds. The host material is preferably a charge transporting material, and one or two or more host materials may be used. For example, the constitution of the mixture of an electron transporting host material and a hole transporting host material is exemplified. Further, a material not having an electron transporting property and not emitting light may be contained in the light-emitting layer.

The light-emitting layer may comprise one layer, or may be two or more layers, and each layer may emit light in different luminescent color.

The examples of fluorescent materials that can be used in the invention include various metal complexes represented by metal complexes of benzoxazole derivatives, benzimidazole derivatives, benzothiazole derivatives, styrylbenzene derivatives, polyphenyl derivatives, diphenylbutadiene derivatives, tetraphenylbutadiene derivatives, naphthalimide derivatives, coumarin derivatives, condensed aromatic compounds, perinone derivatives, oxadiazole derivatives, oxazine derivatives, aldazine derivatives, pyraridine derivatives, cyclopentadiene derivatives, bisstyryl- anthracene derivatives, quinacridone derivatives, pyrrolo- pyridine derivatives, thiadiazolopyridine derivatives, cyclopentadiene derivatives, styrylamine derivatives, diketopyrrolopyrrole derivatives, aromatic dimethylidyne compounds, 8-quinolinol derivatives, and pyrromethene derivatives, polymer compounds such as polythiophene, polyphenylene, polyphenylenevinylene, and compounds such as organic silane derivatives.

The examples of phosphorescent materials that can be used in the invention include complexes containing a transition metal atom or a lanthanoid atom.

The transition metal atoms are not especially restricted, but ruthenium, rhodium, palladium, tungsten, rhenium, osmium, iridium and platinum are preferably exemplified, and rhenium, iridium and platinum are more preferred.

It is preferred to contain a platinum phosphorescent material, and more preferably a platinum complex having a tetradentate ligand.

As lanthanoid atoms, lanthanum, cerium, praseodymium, neodymium, samarium, europium, gadolinium, terbium, dysprosium, holmium, erbium, thulium, ytterbium, and lutecium are exemplified. Of these lanthanoid atoms, neodymium, europium and gadolinium are preferred.

As the examples of ligands of complexes, the ligands described, for example, in G. Wilkinson et al., Comprehensive Coordination Chemistry, Pergamon Press (1987), H. Yersin, Photochemistry and Photophysics of Coordination Compounds, Springer-Verlag (1987), and Akio Yamamoto, Yuki Kinzoku Kagaku -Kiso to Oyo-(Organic Metal Chemistry -Elements and Applications), Shokabo Publishing Co. (1982) are exemplified.

As the specific examples of ligands, halogen ligands (preferably a chlorine ligand), nitrogen-containing heterocyclic ligands (e.g., phenylpyridine, benzoquinoline, quinolinol, bipyridyl, phenanthroline, etc.), diketone ligands (e.g., acetylacetone, etc.), carboxylic acid ligands (e.g., acetic acid ligand, etc.), carbon monoxide ligands, isonitrile ligands, and cyano ligands are preferably exemplified, and more preferably nitrogen-containing heterocyclic ligands. These complexes may have one transition metal atom in a compound, or may be the so-called polynuclear complexes having two or more transition metal atoms. They may contain metal atoms of different kinds at the same time.

It is preferred for a phosphorescent material to be contained in the light-emitting layer in an amount of from 0.1 to 40 mass%, and more preferably from 0.5 to 20 mass%.

As the platinum complex phosphorescent materials having a tetradentate ligand, the compounds disclosed in U.S. Patent 6,653,654, WO 04/108857, WO 04/081017, WO 05/042444, JP-A-2006-232784, WO 05/042550, JP-A-2005-310733, JP-A-2005-317516, JP-A-2006-261623 and WO 06/098505 are specifically exemplified.

As host materials to be contained in the light-emitting layer in the invention, e.g., materials having a carbazole skeleton, having a diarylamine skeleton, having a pyridine skeleton, having a pyrazine skeleton, having a triazine skeleton, having an arylsilane skeleton, and those described later in the items of a hole injecting layer, a hole transporting layer, an electron injecting layer, and an electron transporting layer are exemplified. As the host material, a compound having an indole skeleton is preferred, a compound represented by formula (1) is more preferred, a compound represented by formula (2) or (3) is still more preferred, and a compound represented by formula (4) or (5) is especially preferred.

The thickness of the light-emitting layer is not especially limited, but is generally preferably from 1 to 500 nm, more preferably from 5 to 200 nm, and still more preferably from 10 to 100 nm.

### Hole injecting layer and hole transporting layer:

The hole injecting layer and the hole transporting layer are layers having a function to receive positive holes from the anode or anode side and transport the positive holes to the cathode side. The hole injecting layer and the hole transporting layer are specifically preferably the layers containing carbazole derivatives, azacarbazole derivatives, indole derivatives, azaindole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidyne compounds, porphyrin compounds, organic silane derivatives, carbon, various kinds of metal complexes represented by Ir complex having phenylazole, or phenylazine as the ligand.

An electron accepting dopant can be contained in the positive hole injecting layer or positive hole transporting layer of the organic EL device of the invention. As the electron accepting dopants to be introduced to the hole injecting layer or hole transporting layer, inorganic compounds and organic compounds can be used so long as they are electron accepting and have a property of capable of oxidizing an organic compound.

Specifically, as the inorganic compounds, halogenated metals, e.g., ferric chloride, aluminum chloride, gallium chloride, indium chloride, antimony pentachloride, etc., and metal oxides, e.g., vanadium pentoxide, molybdenum trioxide, etc., are exemplified.

When dopants are organic compounds, the compounds having as a substituent a nitro group, halogen, a cyano group, or a trifluoromethyl group, quinone compounds, acid anhydride compounds, and fullerene are preferably used.

Besides the above compounds, the compounds disclosed in JP-A-6-212153, JP-A-11-111463, JP-A-11-251067, JP-A-2000-196140, JP-A-2000-286054, JP-A-2000-315580, JP-A-2001-102175, JP-A-2001-160493, JP-A-2002-252085, JP-A-2002-56985, JP-A-2003-157981, JP-A-2003-217862, JP-A-2003-229278, JP-A-2004-342614, JP-A-2005-72012, JP-A-2005-166637 and JP-A-2005-209643 can be preferably used.

Of these compounds, hexacyanobutadiene, hexacyanobenzene, tetracyanoethylene, tetracyanoquinodimethane, tetrafluorotetracyanoquinodimethane, p-fluoranyl, p-chloranyl, p-bromanyl, p-benzoquinone, 2,6-dichlorobenzoquinone, 2,5-dichlorobenzoquinone, 1,2,4,5-tetracyanobenzene, 1,4-dicyanotetrafluorobenzene, 2,3-dichloro-5,6-dicyanobenzoquinone, p-dinitrobenzene, m-dinitrobenzene, o-dinitrobenzene, 1,4-naphthoquinone, 2,3-dichloronaphthoquinone, 1,3-dinitronaphthoquinone, 1,5-dinitronaphthalene, 9,10-anthraquinone, 1,3,6,8-tetranitrocarbazole, 2,4,7-trinitro-9-fluorenone, 2,3,5,6-tetracyanopyridine, and fullerene C₆₀ are preferred, hexacyanobutadiene, hexacyanobenzene, tetracyanoethylene, tetracyanoquinodimethane, tetrafluorotetracyanoquinodimethane, p-fluoranyl, p-chloranyl, p-bromanyl, 2,6-dichlorobenzoquinone, 2,5-dichlorobenzoquinone, 2,3-dichloronaphthoquinone, 1,2,4, 5-tetracyanobenzene, 2,3-dichloro-5,6-dicyanobenzoquinone, and 2,3,5,6-tetracyanopyridine are more preferred, and tetrafluorotetracyanoquinodimethan is especially preferred.

These electron accepting dopants may be used by one kind alone, or two or more kinds may be used in combination. The amount of the electron accepting dopant to be used differs according to the kind of the material, but the amount is preferably from 0.01 to 50 mass% to the material of the positive hole transporting layer, more preferably from 0.05 to 20 mass%, and still more preferably from 0.1 to 10 mass%.

The thickness of the hole injecting layer and hole transporting layer is preferably 500 nm or less from the viewpoint of lowering driving voltage.

The thickness of the hole transporting layer is preferably from 1 to 500 nm, more preferably from 5 to 200 nm, and still more preferably from 10 to 100 nm. The thickness of the hole injecting layer is preferably from 0.1 to 200 nm, more preferably from 0.5 to 100 nm, and still more preferably from 1 to 100 nm.

The hole injecting layer and the hole transporting layer may be a single layer structure comprising one or two or more of the above materials, or may be a multilayer structure comprising a plurality of layers of the same or different compositions.

### Electron injecting layer and electron transporting layer:

The electron injecting layer and the electron transporting layer are layers having a function to receive electrons from the cathode or cathode side and transport the electrons to the anode side. The electron injecting layer and the electron transporting layer are specifically preferably layers containing triazole derivatives, oxazole derivatives, oxadiazole derivatives, imidazole derivatives, fluorenone derivatives, anthraquinodimethane derivatives, anthrone derivatives, diphenylquinone derivatives, thiopyran dioxide derivatives, carbodiimide derivatives, fluorenylidenemethane derivatives, distyrylpyrazine derivatives, tetracarboxylic anhydride of aromatic rings such as naphthalene, perylene, etc., a phthalocyanine derivative, various metal complexes represented by metal complexes of 8-quinolinol derivatives, metalphthalocyanine, metal complexes having benzoxazole, benzothiazole as the ligand, organic silane derivative, etc.

An electron donating dopant can be contained in the electron injecting layer or electron transporting layer of the organic EL elemental device of the invention. Any compound can be used as the electron donating dopant to be introduced to the electron injecting layer or electron transporting layer, so long as the compound is electron accepting and has a property of capable of reducing an organic compound, and alkali metal salts, e.g., Li, alkaline earth metals, e.g., Mg, transition metals containing a rare earth metal, and reducing organic compounds are preferably used as the electron donating dopant. As the metals, metals having a work function of 4.2 eV or less can be preferably used, and specifically Li, Na, K, Be, Mg, Ca, Sr, Ba, Y, Cs, La, Sm, Gd and Yb are exemplified. As the reducing organic compounds, e.g., nitrogen-containing compounds, sulfur-containing compounds, and phosphorus- containing compounds are exemplified.

Besides the above, the materials disclosed in JP-A-6-212153, JP-A-2000-196140, JP-A-2003-68468, JP-A-2003-229278, and JP-A-2004-342614 can be used.

These electron donating dopants may be used alone, or two or more kinds may be used in combination. The use amount of the electron donating dopants differs according to the kind of the material, but the amount is preferably from 0.1 to 99 mass% to the material of the electron transporting layer, more preferably from 1.0 to 80 mass%, and especially preferably from 2.0 to 70 mass%.

The thickness of each of the electron injecting layer and electron transporting layer is preferably 500 nm or less from the viewpoint of lowering driving voltage.

The thickness of the electron transporting layer is preferably from 1 to 500 nm, more preferably from 5 to 200 nm, and still more preferably from 10 to 100 nm. The thickness of the electron injecting layer is preferably from 0.1 to 200 nm, more preferably from 0.2 to 100 nm, and still more preferably from 0.5 to 50 nm.

The electron injecting layer and the electron transporting layer may be a single layer structure comprising one or two or more of the above materials, or may be a multilayer structure comprising a plurality of layers of the same or different compositions.

### Hole blocking layer:

A hole blocking layer is a layer having a function of preventing the positive holes transported from the anode side to the light-emitting layer from passing through to the cathode side. In the invention, a hole blocking layer can be provided as the organic layer contiguous to the light-emitting layer on the cathode side.

As the examples of the organic compounds constituting the hole blocking layer, aluminum complexes, e.g., aluminum (III) bis(2-methyl-8-quinolinato)-4-phenylphenolate (abbreviated to BAlq), etc., triazole derivatives, phenanthroline derivatives, e.g., 2,9-dimethyl-4,7-diphenyl- 1,10-phenanthroline (abbreviated to BCP), etc., are exemplified.

The thickness of the hole blocking layer is preferably from 1 to 500 nm, more preferably from 5 to 200 nm, and still more preferably from 10 to 100 nm.

The hole blocking layer may be a single layer structure comprising one or two or more of the above materials, or may be a multilayer structure comprising a plurality of layers of the same or different compositions.

### Protective layer:

In the invention the organic EL device may be completely protected with a protective layer.

It is sufficient for the materials to be contained in the protective layer to have a function capable of restraining the substances accelerating deterioration of device, e.g., water, oxygen, etc., from entering the device.

The specific examples of such materials include metals, e.g., In, Sn, Pb, Au, Cu, Ag, Al, Ti, Ni, etc., metal oxides, e.g., MgO, SiO, SiO₂, Al₂O₃, GeO, NiO, CaO, BaO, Fe₂O₃, Y₂O₃, TiO₂, etc., metal nitrides, e.g., SiNₓ, SiNₓOy, etc., metal fluorides, e.g., MgF₂, LiF, AlF₃, CaF₂, etc., polyethylene, polypropylene, polymethyl methacrylate, polyimide, polyurea, polytetrafluoroethylene, polychlorotrifluoroethylene, polydichlorodifluoroethylene, copolymers of chlorotrifluoro- ethylene with dichlorodifluoroethylene, copolymers obtained by copolymerization of a monomer mixture containing tetrafluoroethylene and at least one comonomer, fluorine- containing copolymers having a cyclic structure on the main chain of the copolymer, water absorptive substances having a water absorption rate of not lower than 1%, moisture proofing substances having a water absorption rate of not higher than 0.1%.

The forming method of the protective layer is not especially restricted and, for example, a vacuum deposition method, a sputtering method, a reactive sputtering method, an MBE (molecular beam epitaxy) method, a cluster ion beam method, an ion plating method, a plasma polymerization method (a high frequency excitation ion plating method), a plasma CVD method, a laser CVD method, a heat CVD method, a gas source CVD method, a coating method, a printing method, a transfer method, etc., can be applied to the invention.

### Sealing:

The organic electroluminescent device of the invention may be completely sealed in a sealing container.

Further, a water absorber or an inert liquid may be filled in the space between the sealing container and the light-emitting device. The water absorber is not especially restricted and, for example, barium oxide, sodium oxide, potassium oxide, calcium oxide, sodium sulfate, calcium sulfate, magnesium sulfate, phosphorus pentoxide, calcium chloride, magnesium chloride, copper chloride, cesium fluoride, niobium fluoride, calcium bromide, vanadium bromide, molecular sieve, zeolite, magnesium oxide, etc., can be exemplified. The inert liquid is not particularly limited and, for example, paraffins, liquid paraffins, fluorine solvents, such as perfluoroalkane, perfluoroamine, perfluoroether, etc., chlorine solvents, and silicone oils are exemplified.

Luminescence can be obtained by the application of DC (if necessary, an alternating current factor may be contained) voltage (generally from 2 to 15 V) or DC electric current between the anode and cathode of the organic electroluminescent device of the invention.

In connection with the driving methods of the organic electroluminescent device of the invention, the driving methods disclosed in JP-A-2-148687, JP-A-6-301355, JP-A-5-29080, JP-A-7-134558, JP-A-8-234685, JP-A-8-241047, Japanese Patent 2784615, and U.S. Patents 5,828,429 and 6,023,308 can be used.

### Examples

The invention will be described more specifically with reference to examples, but the invention should not be construed as being restricted thereto.

### Synthesis of Exemplified Compound (1-3):

Under the nitrogen atmosphere, a mixed solution comprising 11.92 g (50 mmol) of 2-chloro-1-iodobenzene, 12.2 ml (100 mmol) of 3,3-dimethylbutyne, 350 mg (0.5 mmol) of dichlorobis(triphenylphosphine)palladium, 95 mg (0.5 mmol) of copper(I) iodide, and 100 ml of triethylamine is stirred by heating while refluxing for 1 hour. The obtained reaction solution is concentrated, and the resulting residue is refined with silica gel column chromatography to obtain 9.42 g (49 mmol, yield: 98%) of (2-chlorophenyl)-3,3-dimethylbutyne.

Under the nitrogen atmosphere, a mixed solution comprising 5.34 g (27.7 mmol) of (2-chlorophenyl)-3,3- dimethylbutyne, 1.50 g (13.9 mmol) of 1,3-diaminobenzene, 291 mg (1.3 mmol) of palladium acetate, 553 mg (1.3 mmol) of 1,3-bis(2,6-diisopropylphenyl)imidazolium chloride, 9.30 g (83 mmol) of potassium tert-butoxylate, and 100 ml of xylene is stirred by heating while refluxing for 4 hours. Water is added to the obtained reaction solution, an organic layer extracted with ethyl acetate is dried with magnesium sulfate and concentrated under reduced pressure. The resulting residue is refined with silica gel column chromatography and recrystallized to obtain 7.27 g (10.17 mmol, yield: 73%) of exemplified compound (1-3).
¹H-NMR of exemplified compound (1-3): 1.34 (d, 18H), 6.49 (d, 2H), 6.70 (dd, 2H), 7.03-7.15 (m, 4H), 7.44 (t, 1H), 7.48-7.60 (m, 4H), 7.63-7.69 (m, 1H) 400 MHz

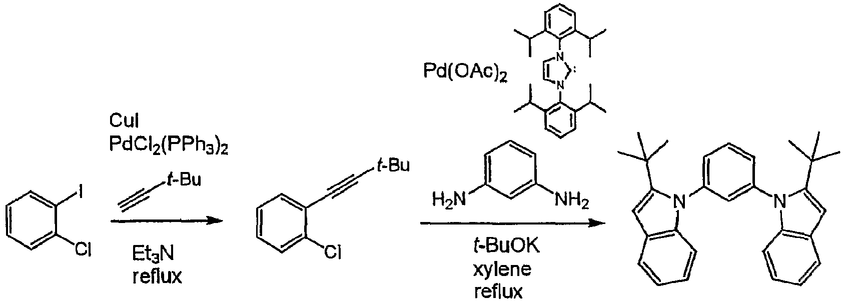

### Synthesis of Compound (1-8):

Compound (1-8) was obtained using 1,4-diaminobenzene in place of 1,3-diaminobenzene in Synthesis Example of Compound (1-3).

### Compound (1-8):

¹H-NMR: 1.33 (s, 8.1H), 1.37 (s, 9.9H), 6.50-6.56 (m, 2H), 6.66-6.74 (m, 0.9H), 6.77-6.85 (m, 1.1H), 7.05-7.18 (m, 4H), 7.50-7.54 (m, 4H), 7.57-7.65 (m, 2H) 300 MHz.

Other compounds of the invention can also be synthesized according to similar methods. For example, compound (1-8) can be synthesized by using 1,4-diaminobenzene in place of 1,3-diaminobenzene.

### COMPARATIVE EXAMPLE 1

A cleaned ITO substrate is placed in a vacuum evaporator, copper phthalocyanine is deposited on the substrate in a thickness of 10 nm, and NPD (N,N'-di-α-naphthyl-N,N'-diphenyl)benzidine is deposited thereon in a thickness of 40 nm. Compound B-1 and compound A in the ratio of 12/88 (by mass) are deposited on the above deposited film in a thickness of 30 nm, then BAlq is deposited thereon in a thickness of 6 nm, and then Alq (tris(8-hydroxyquinoline) aluminum complex) is deposited on the above film in a thickness of 20 nm. Lithium fluoride is deposited thereon in a thickness of 3 nm, followed by deposition of aluminum in a thickness of 60 nm to prepare an elemental device. The obtained EL elemental device is subjected to application of DC constant voltage with a source measure unit Model 2400 (manufactured by Toyo Technica Co., Ltd.) to emit luminescence. It is confirmed that the emission of phosphorescence originating in compound B-1 is obtained.

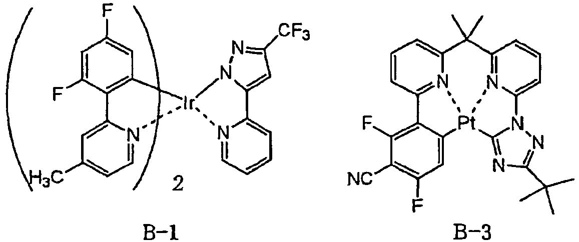

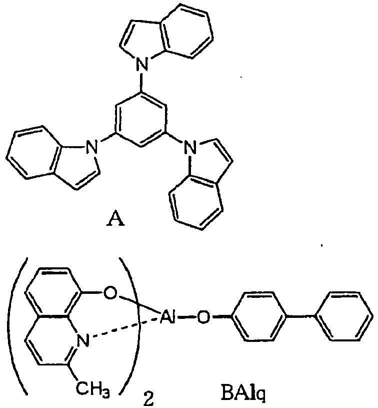

### EXAMPLE 1 (Reference)

Evaluation of a device is performed in the same manner as in Comparative Example 1 except for using exemplified compound (1-1) of the invention in place of compound A. It is confirmed that the emission of blue phosphorescence originating in compound B-1 is obtained.

### EXAMPLE 2

Evaluation of a device is performed in the same manner as in Example 1 except for using exemplified compound (1-8) in place of compound (1-1). It is confirmed that the emission of phosphorescence originating in compound B-1 is obtained.

### EXAMPLE 3 (Reference)

Evaluation of a device is performed in the same manner as in Example 1 except for using compound B-3 in place of compound B-1. It is confirmed that the emission of blue phosphorescence originating in compound B-3 is obtained.

### EXAMPLE 4

Evaluation of a device is performed in the same manner as in Example 2 except for using compound B-3 in place of compound B-1. It is confirmed that the emission of blue phosphorescence originating in compound B-3 is obtained.

### EXAMPLE 5 (Reference)

Evaluation of a device is performed in the same manner as in Example 3 except for inserting a layer containing exemplified compound (1-8) having a thickness of 3 nm between the NPD layer and the light-emitting layer. It is confirmed that the emission of phosphorescence originating in compound B-3 is obtained.

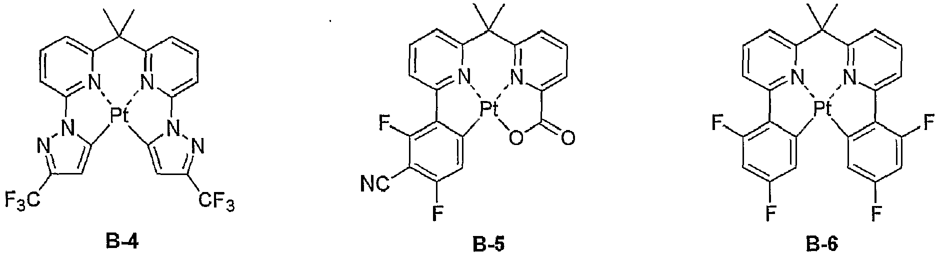

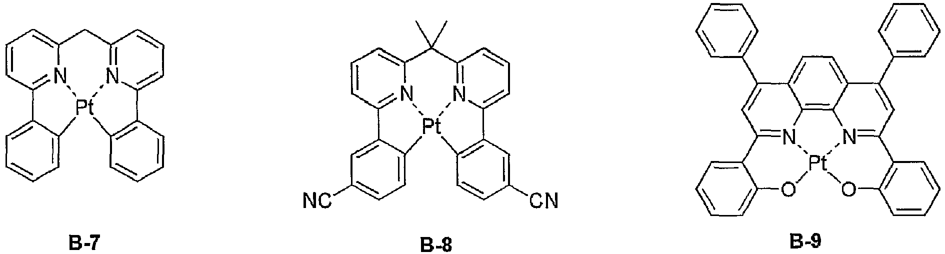

### EXAMPLE 6 to EXAMPLE 13

Devices were produced and evaluated in the same manner as in Example 2 by using (1-3) in place of (1-8) and using B-4 to B-9 in place of B-1 in Example 2. Phosphorescence derived from each light-emitting material was obtained. The materials used in place of B-1 are shown in Table 2.

### COMPARATIVE EXAMPLE 2

A device was produced in the same manner as in Comparative Example 1 by changing the composition of the light-emitting layer to a rubrene (fluorescent material) and Compound A (host material) at a ratio (by mass) of 1:99, and the device was evaluated. Fluorescence derived from rubrene was obtained.

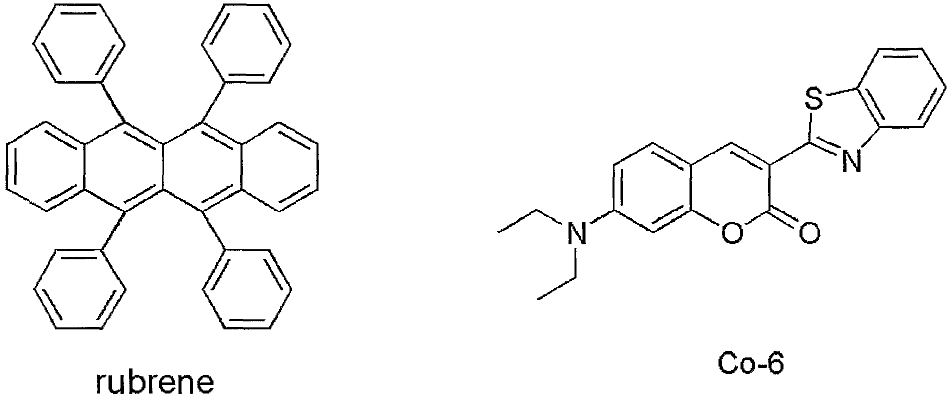

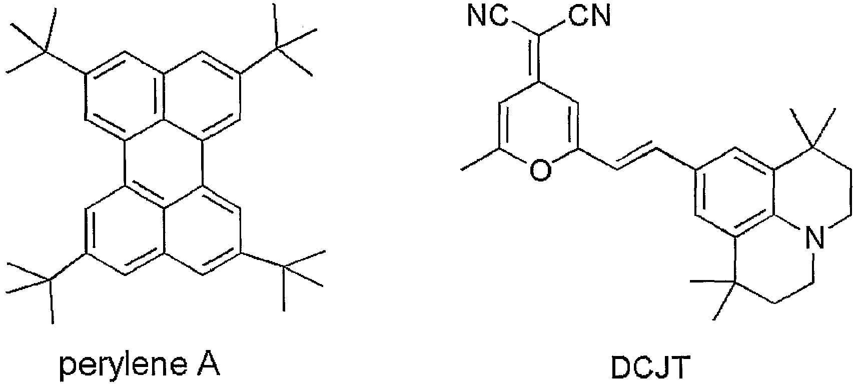

### COMPARATIVE EXAMPLES 3 to 5 and EXAMPLES 14 to 21

Devices were produced in the same manner by changing the fluorescent material and the host material in Comparative Example 2, and the elements were evaluated. Fluorescence derived from rubrene was obtained. The construction of the light-emitting layer of each element is shown in Table 3.

### EXAMPLE 22

A device where Compound 1-8 was inserted in a thickness of 1 nm between the light-emitting layer and the Balq layer of Comparative Example 5 was produced, and the device was evaluated in the same manner.

### EXAMPLE 23

A device where Compound 1-8 was inserted in a thickness of 1 nm between the light-emitting layer and the NPD layer of Comparative Example 5 was produced, and the device was evaluated in the same manner.

### Evaluation of light-emitting device:

Each of the obtained light-emitting devices is driven at 20 °C by the application of constant electric current. Luminance is measured with a luminance meter BM-8 (trade name, manufactured by Topcon Co.). Emission spectrum is measured with an emission spectrum measuring system (ELS1500, manufactured by Shimadzu Corporation). The half life time of luminance is found by measuring the time required to reach the half life of luminance from the initial luminance of 360 cd/m². CIE Y value is found from the emission spectrum measured at 20 °C with an emission spectrum measuring system (ELS1500, manufactured by Shimadzu Corporation), and the variation in chromaticity is computed from the CIE Y value. The light-emitting device is driven at 20 °C and luminance of 360 cd/m² by the application of constant current, and the external quantum efficiency is computed from the obtained emission spectrum and front luminance by a luminance conversion method.

The results obtained are shown in Table 1 below.

**TABLE 1**

| Results of evaluation of devices prepared in Comparative Example 1 and Examples 1 to 5 | | | |
|---|---|---|---|
| Example No. | External Quantum Efficiency (relative value) | Half Life Time of Luminance (relative value) | Variation in Chromaticity at Half Life Time of Luminance (rate of change of CIE y value in relative value) |
| Comparative Example 1 | 1.0 | 1.0 | 1.0 |
| Example 1 Reference | 2.5 | 1.7 | 0.2 |
| Example 2 | 2.2 | 1.5 | 0.2 |
| Example 3 Reference | 2.4 | 2.6 | 0.2 |
| Example 4 | 2.1 | 2.0 | 0.2 |
| Example 5 Reference | 2.6 | 2.9 | 0.2 |

| | | | |
|---|---|---|---|
| * The evaluation value in each example is a relative value to the evaluation value of Comparative Example 1 as the reference value. | | | |

**TABLE 2**

| Results of evaluation of devices prepared in Examples 6 to 13 | | | | |
|---|---|---|---|---|
| Example No. | External Quantum Efficiency (relative value) | Half Life Time of Luminance (relative value) | Variation in Chromaticity at Half Life Time of Luminance (rate of change of CIE y value in relative value) | Phosphorescence material |
| Example 6 | 2.7 | 2.8 | 0.3 | B-1 |
| Example 7 | 2.8 | 3.1 | 0.2 | B-3 |
| Example 8 | 2.4 | 2.8 | 0.1 | B-4 |
| Example 9 | 2.5 | 2.9 | 0.2 | B-5 |
| Example 10 | 2.7 | 3.4 | 0.1 | B-6 |
| Example 11 | 2.6 | 3.1 | 0.2 | B-7 |
| Example 12 | 2.5 | 3.0 | 0.2 | B-8 |
| Example 13 | 2.7 | 2.6 | 0.3 | B-9 |

| | | | | |
|---|---|---|---|---|
| * The evaluation value in each example is a relative value to the evaluation value of Comparative Example 1 as the reference value. | | | | |

**TABLE 3**

| | External Quantum Efficiency (relative value) | Half Life Time of Luminance (relative value) | Variation in Chromaticity at Half Life Time of Luminance (rate of change of CIE y value in relative value) | Construction of Light-Emitting Layer | |
|---|---|---|---|---|---|
| | | | | Fluorescent Material | Host Material |
| Comparative Example 2 | 0.3 | 0.7 | 1.0 | rubrene | A |
| Comparative Example 3 | 0.4 | 0.5 | 1.2 | Co-6 | A |
| Comparative Example 4 | 0.3 | 0.3 | 1.4 | perylene A | A |
| Comparative Example 5 | 0.2 | 0.4 | 1.1 | DCJT | A |
| Example 14 | 0.7 | 1.5 | 0.6 | rubrene | 1-3 |
| Example 15 | 0.7 | 1.0 | 0.7 | Co-6 | 1-3 |
| Example 16 | 0.6 | 0.8 | 0.9 | perylene A | 1-3 |
| Example 17 | 0.7 | 0.8 | 0.8 | DCJT | 1-3 |
| Example 18 | 0.6 | 1.3 | 0.6 | rubrene | 1-8 |
| Example 19 | 0.8 | 1.0 | 0.3 | Co-6 | 1-8 |
| Example 20 | 0.7 | 0.8 | 0.7 | perylene A | 1-8 |
| Example 21 | 0.5 | 0.9 | 0.5 | DCJT | 1-8 |
| Example 22 | 0.6 | 0.8 | 0.7 | DCJT | A |
| Example 23 | 0.5 | 0.9 | 0.6 | DCJT | A |

| | | | | | |
|---|---|---|---|---|---|
| * The evaluation value in each example is a relative value to the evaluation value of Comparative Example 1 as the reference value. | | | | | |

From the results in Tables 1 to 3, it can be seen that the luminescent elemental devices in the invention are excellent in efficiency and durability, and little in chromaticity variation.

The same effects can be obtained with luminescent elemental devices using other compounds according to the invention.

## Claims

1. An organic electroluminescent device comprising:
a pair of electrodes; and
at least one organic layer between the pair of electrodes, the at least one organic layer including a light-emitting layer containing a light-emitting material,
wherein the at least one organic layer includes at least one layer containing an indole derivative represented by formula (1) : 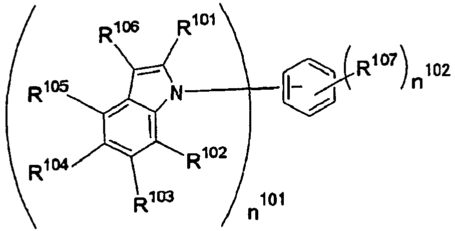 wherein R¹⁰², R¹⁰³, R¹⁰⁴, R¹⁰⁵ and R¹⁰⁶ each independently represents a hydrogen atom or a substituent; R¹⁰¹ represents an alkyl group having a tertiary or quaternary carbon atom; R¹⁰¹ and R¹⁰⁶ may be bonded to each other to form a ring; R¹⁰⁷ represents a substituent; n¹⁰¹ represents 2; and n¹⁰² represents an integer of from 0 to 5, provided that n¹⁰¹ + n¹⁰² ≤ 6.

2. The organic electroluminescent device according to claim 1, wherein R¹⁰¹ represents a t-butyl group, an isopropyl group, an isobutyl group, an isopentyl group, a cyclopropyl group, a cyclopentyl group, a cyclohexyl group, a 2-phenyl-2-propyl group, or a 2-(2-pyridyl)propyl group.

3. The organic electroluminescent device according to claim 1, wherein R¹⁰¹ represents a t-alkyl group.

4. The organic electroluminescent device according to claim 1, wherein the indole derivative is a compound represented by formula (2): 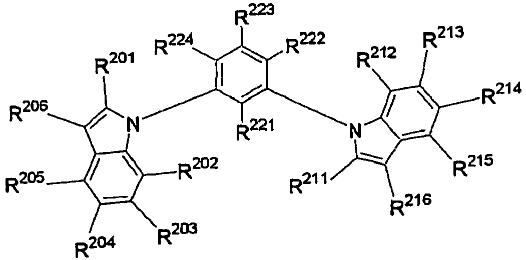 wherein R²⁰², R²⁰³, R²⁰⁴, R²⁰⁵ and R²⁰⁶ each independently represents a hydrogen atom or a substituent; R²⁰¹ represents an alkyl group having a tertiary or quaternary carbon atom; R²⁰¹ and R²⁰⁶ may be bonded to each other to form a ring; R²¹², R²¹³, R²¹⁴, R²¹⁵ and R²¹⁶ each independently represents a hydrogen atom or a substituent; R²¹¹ represents an alkyl group having a tertiary or quaternary carbon atom; R²¹¹ and R²¹⁶ may be bonded to each other to form a ring; and R²²¹, R²²², R²²³ and R²²⁴ each independently represents a hydrogen atom or a substituent linking via a carbon atom.

5. The organic electroluminescent device according to claim 4, wherein R²⁰¹ and R¹¹¹ each independently represents a t-butyl group, an isopropyl group, an isobutyl group, an isopentyl group, a cyclopropyl group, a cyclopentyl group, a cyclohexyl group, a 2-phenyl-2-propyl group, or a 2-(2-pyridyl)propyl group.

6. The organic electroluminescent device according to claim 1, wherein the indole derivative is a compound represented by formula (3): 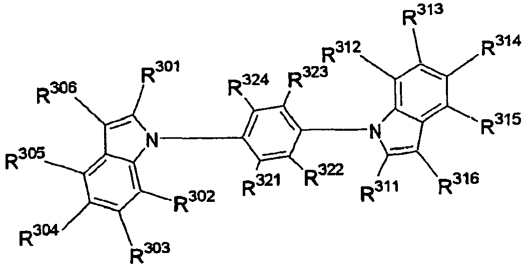 wherein R³⁰², R³⁰³, R³⁰⁴, R³⁰⁵ and R³⁰⁶ each independently represents a hydrogen atom or a substituent; R³⁰¹ represents an alkyl group having a tertiary or quaternary carbon atom; R³⁰¹ and R³⁰⁶ may be bonded to each other to form a ring_{;} R³¹², R³¹³, R³¹⁴, R³¹⁵ and R³¹⁶ each independently represents a hydrogen atom or a substituent; R³¹¹ represents an alkyl group having a tertiary or quaternary carbon atom; R³¹¹ and R³¹⁶ may be bonded to each other to form a ring; and R³²¹, R³²², R³²³ and R³²⁴ each independently represents a hydrogen atom or a substituent linking via a carbon atom.

7. The organic electroluminescent device according to claim 6, wherein R³⁰¹ and R³¹¹ each independently represents a t-butyl group, an isopropyl group, an isobutyl group, an isopentyl group, a cyclopropyl group, a cyclopentyl group, a cyclohexyl group, a 2-phenyl-2-propyl group, or a 2-(2-pyridyl)propyl group.

8. The organic electroluminescent device according to any one of claims 1 to 2, wherein the indole derivative is a compound represented by formula (4): 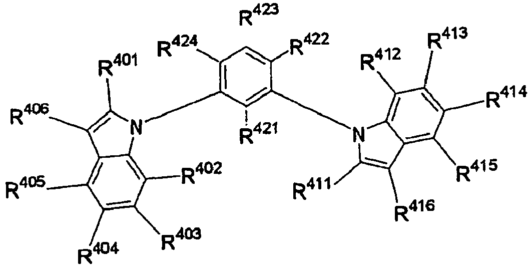 wherein R⁴⁰², R⁴⁰³, R⁴⁰⁴, R⁴⁰⁵ and R⁴⁰⁶ each independently represents a hydrogen atom or a substituent; R⁴⁰¹ represents a t-alkyl group; R⁴⁰¹ and R⁴⁰⁶ may be bonded to each other to form a ring; R⁴¹², R⁴¹³, R⁴¹⁴, R⁴¹⁵ and R⁴¹⁶ each independently represents a hydrogen atom or a substituent; R⁴¹¹ represents a t-alkyl group; R⁴¹¹ and R⁴¹⁶ may be bonded to each other to form a ring; and R⁴²¹, R⁴²², R⁴²³ and R⁴²⁴ each independently represents a hydrogen atom or a substituent linking via a carbon atom.

9. The organic electroluminescent device according to any one of claims 1 to 2, wherein the indole derivative is a compound represented by formula (5): 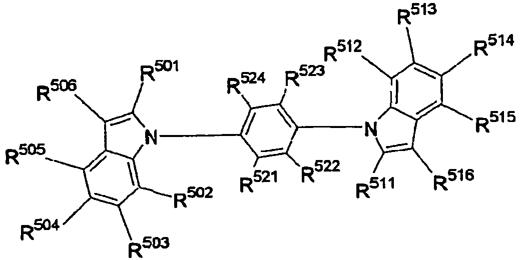 wherein R⁵⁰², R⁵⁰³, R⁵⁰⁴, R⁵⁰⁵ and R⁵⁰⁶ each independently represents a hydrogen atom or a substituent; R⁵⁰¹ represents a t-alkyl group; R⁵⁰¹ and R⁵⁰⁶ may be bonded to each other to form a ring; R⁵¹², R⁵¹³, R⁵¹⁴, R⁵¹⁵ and R⁵¹⁶ each independently represents a hydrogen atom or a substituent; R⁵¹¹ represents a t-alkyl group; R⁵¹¹ and R⁵¹⁶ may be bonded to each other to form a ring; and R⁵²¹, R⁵²², R⁵²³ and R⁵²⁴ independently each represents a hydrogen atom or a substituent linking via a carbon atom.

10. The organic electroluminescent according to any one of claims 1 to 9, wherein the indole derivative is contained in the light-emitting layer.

11. The organic electroluminescent device according to any one of claims 1 to 10, wherein the indole derivative is contained in a layer contiguous to the light-emitting layer.

12. A compound represented by formula (4): 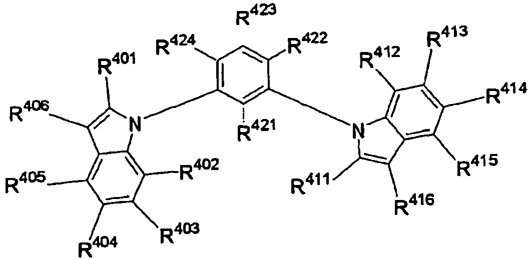 wherein R⁴⁰², R⁴⁰³, R⁴⁰⁴, R⁴⁰⁵ and R⁴⁰⁶ each independently represents a hydrogen atom or a substituent; R⁴⁰¹ represents a t-alkyl group; R⁴⁰¹ and R⁴⁰⁶ may be bonded to each other to form a ring; R⁴¹², R⁴¹³, R⁴¹⁴, R⁴¹⁵ and R⁴¹⁶ each independently represents a hydrogen atom or a substituent; R⁴¹¹ represents a t-alkyl group; R⁴¹¹ and R⁴¹⁶ may be bonded to each other to form a ring; and R⁴²¹, R⁴²², R⁴²³ and R⁴²⁴ each independently represents a hydrogen atom or a substituent linking via a carbon atom.

13. A compound represented by formula (5): 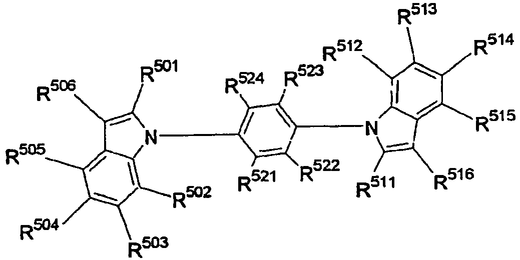 wherein R⁵⁰², R⁵⁰³, R⁵⁰⁴, R⁵⁰⁵ and R⁵⁰⁶ each independently represents a hydrogen atom or a substituent; R⁵⁰¹ represents a t-alkyl group; R⁵⁰¹ and R⁵⁰⁶ may be bonded to each other to form a ring; R⁵¹², R⁵¹³, R⁵¹⁴, R⁵¹⁵ and R⁵¹⁶ each independently represents a hydrogen atom or a substituent; R⁵¹¹ represents a t-alkyl group; R⁵¹¹ and R⁵¹⁶ may be bonded to each other to form a ring; and R⁵²¹, R⁵²², R⁵²³ and R⁵²⁴ independently each represents a hydrogen atom or a substituent linking via a carbon atom.

## Patentansprüche

1. Eine organische elektrolumineszente Vorrichtung umfassend:
Ein Paar von Elektroden; und
mindestens eine organische Schicht zwischen dem Paar von Elektroden, die mindestens eine organische Schicht umfassend eine Licht emittierende Schicht enthaltend ein Licht emittierendes Material, wobei die mindestens eine organische Schicht mindestens eine Schicht umfasst, welche ein Indolderivat enthält, wiedergegeben durch Formel (1): 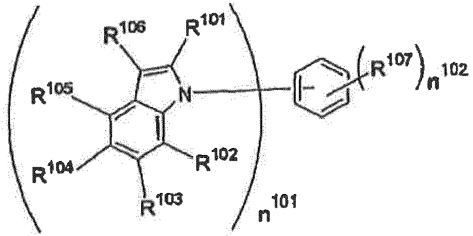 wobei R¹⁰², R¹⁰³, R¹⁰⁴, R¹⁰⁵ und R¹⁰⁶ jeweils unabhängig für ein Wasserstoffatom oder einen Substituenten stehen; R¹⁰¹ steht für eine Alkylgruppe mit einem tertiären oder quaternären Kohlenstoffatom; R¹⁰¹ und R¹⁰⁶ können aneinander gebunden sein, um einen Ring zu bilden; R¹⁰⁷ steht für einen Substituenten; n¹⁰¹ steht für 2; und n¹⁰² steht für eine ganze Zahl von 0 bis 5, vorausgesetzt, dass n¹⁰¹ + n¹⁰² ≤ 6 ist.

2. Die organische elektrolumineszente Vorrichtung nach Anspruch 1, wobei R¹⁰¹ steht für eine t-Butylgruppe, eine Isopropylgruppe, eine Isobutylgruppe, eine Isopentylgruppe, eine Cyclopropylgruppe, eine Cyclopentylgruppe, eine Cyclohexylgruppe, eine 2-Phenyl-2-propylgruppe oder eine 2-(2-Pyridyl)propylgruppe.

3. Die organische elektrolumineszente Vorrichtung gemäß Anspruch 1, wobei R¹⁰¹ für eine t-Alkylgruppe steht.

4. Die organische elektrolumineszente Vorrichtung gemäß Anspruch 1, wobei das Indolderivat eine Verbindung ist, wiedergegeben durch die Formel (2): 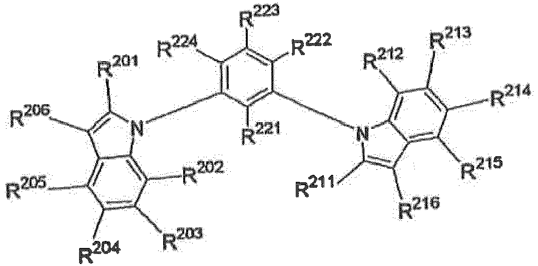 wobei R²⁰², R²⁰³, R²⁰⁴, R²⁰⁵ und R²⁰⁶ jeweils unabhängig für ein Wasserstoffatom oder einen Substituenten stehen; R²⁰¹ steht für eine Alkylgruppe mit einem tertiären oder quaternären Kohlenstoffatom; R²⁰¹ und R²⁰⁶ können aneinander gebunden sein, um einen Ring zu bilden; R²¹², R²¹³, R²¹⁴, R²¹⁵ und R²¹⁶ stehen unabhängig jeweils für ein Wasserstoffatom oder einen Substituenten; R²¹¹ steht für eine Alkylgruppe mit einem tertiären oder quaternären Kohlenstoffatom; R²¹¹ und R²¹⁶ können aneinander gebunden sein, um einen Ring zu bilden; und R²²¹, R²²², R²²³ und R²²⁴ stehen jeweils unabhängig für ein Wasserstoffatom oder einen Substituenten, der über ein Kohlenstoffatom bindet.

5. Die organische elektrolumineszente Vorrichtung gemäß Anspruch 4, wobei R²⁰¹ und R¹¹¹ jeweils unabhängig für eine t-Butylgruppe, eine Isopropylgruppe, eine Isobutylgruppe, eine Isopentylgruppe, eine Cyclopropylgruppe, eine Cyclopentylgruppe, eine Cyclohexylgruppe, eine 2-Phenyl-2-propylgruppe oder eine eine 2-(2-Pyridyl)propylgruppe steht.

6. Die organische elektrolumineszente Vorrichtung gemäß Anspruch 1, wobei das Indolderivat eine Verbindung ist, wiedergegeben durch Formel (3): 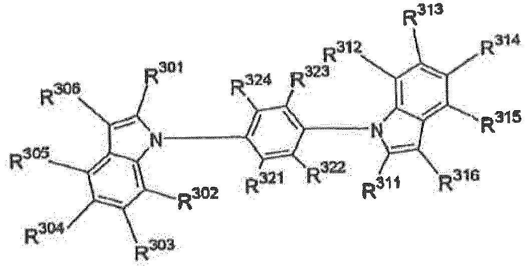 wobei R³⁰², R³⁰³, R³⁰⁴, R³⁰⁵ und R³⁰⁶ jeweils unabhängig für ein Wasserstoffatom oder einen Substituenten stehen; R³⁰¹ steht für eine Alkylgruppe mit einem tertiären oder quaternären Kohlenstoffatom; R³⁰¹ und R³⁰⁶ können aneinander gebunden sein, um einen Ring zu bilden; R³¹², R³¹³, R³¹⁴ , R³¹⁵ und R³¹⁶ stehen jeweils unabhängig für ein Wasserstoffatom oder einen Substituenten; R³¹¹ steht für eine Alkylgruppe mit einem tertiären oder quaternären Kohlenstoffatom; R³¹¹ und R³¹⁶ können aneinander gebunden sein, um einen Ring zu bilden; und R³²¹, R³²², R³²³ und R³²⁴ stehen jeweils unabhängig für ein Wasserstoffatom oder einen Substituenten, der über ein Kohlenstoffatom bindet.

7. Die organische elektrolumineszente Vorrichtung gemäß Anspruch 6, wobei R³⁰¹ und R³¹¹ jeweils unabhängig stehen für eine t-Butylgruppe, eine Isopropylgruppe, eine Isobutylgruppe, eine Isopentylgruppe, eine Cyclopropylgruppe, eine Cyclopentylgruppe, eine Cyclohexylgruppe, eine 2-Phenyl-2-propylgruppe oder eine 2-(2-Pyridyl)propylgruppe.

8. Die organische elektrolumineszente Vorrichtung gemäß einem der Ansprüche 1 bis 2, wobei das Indolderivat eine Verbindung ist, wiedergegeben durch Formel (4): 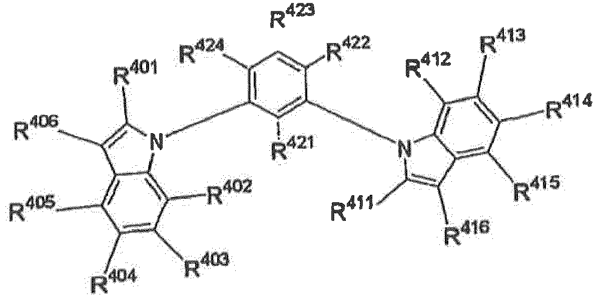 wobei R⁴⁰², R⁴⁰³, R⁴⁰⁴, R⁴⁰⁵ und R⁴⁰⁶ jeweils unabhängig für ein Wasserstoffatom oder einen Substituenten stehen; R⁴⁰¹ steht für eine t-Alkylgruppe; R⁴⁰¹ und R⁴⁰⁶ können aneinander gebunden sein, um einen Ring zu bilden; R⁴¹², R⁴¹³,R⁴¹⁴, R⁴¹⁵ und R⁴¹⁶ stehen jeweils unabhängig für ein Wasserstoffatom oder einen Substituenten; R⁴¹¹ steht für eine t-Alkylgruppe; und R⁴¹¹ und R⁴¹⁶ können aneinander gebunden sein, um einen Ring zu bilden; und R⁴²¹, R⁴²², R⁴²³ und R⁴²⁴ stehen jeweils unabhängig für ein Wasserstoffatom oder einen Substituenten, der über ein Kohlenstoffatom bindet.

9. Die organische elektrolumineszente Vorrichtung gemäß einem der Ansprüche 1 bis 2, wobei das Indolderivat eine Verbindung ist, wiedergegeben durch Formel (5): 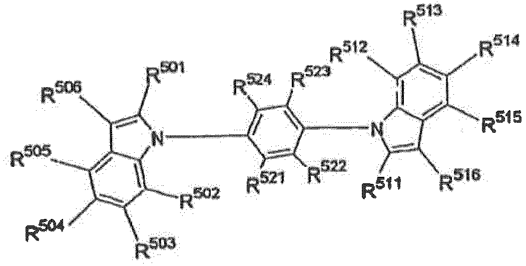 wobei R⁵⁰², R⁵⁰³, R⁵⁰⁴, R⁵⁰⁵ und R⁵⁰⁶ jeweils unabhängig für ein Wasserstoffatom oder einen Substituenten stehen; R⁵⁰¹ steht für eine t-Alkylgruppe; R⁵⁰¹ und R⁵⁰⁶ können aneinander gebunden sein, um einen Ring zu bilden; R⁵¹², R⁵¹³, R⁵¹⁴, R⁵¹⁵ und R⁵¹⁶ stehen jeweils unabhängig für ein Wasserstoffatom oder einen Substituenten; R⁵¹¹ steht für eine t-Alkylgruppe; R⁵¹¹ und R⁵¹⁶ können aneinander gebunden sein, um einen Ring zu bilden; und R⁵²¹, R⁵²², R⁵²³ und R⁵²⁴ stehen jeweils unabhängig für ein Wasserstoffatom oder einen Substituenten, der über ein Kohlenstoffatom bindet.

10. Die organische elektrolumineszente Vorrichtung gemäß einem der Ansprüche 1 bis 9, wobei das Indolderivat in der Licht emittierenden Schicht enthalten ist.

11. Die organische elektrolumineszente Vorrichtung gemäß einem der Ansprüche 1 bis 10, wobei das Indolderivat in einer Schicht angrenzend zur Licht emittierenden Schicht enthalten ist.

12. Eine Verbindung wiedergegeben durch Formel (4): 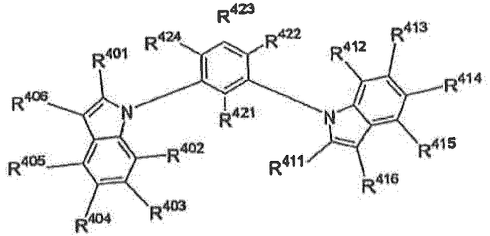 wobei R⁴⁰², R⁴⁰³, R⁴⁰⁴, R⁴⁰⁵ und R⁴⁰⁶ jeweils unabhängig für ein Wasserstoffatom oder einen Substituenten stehen; R⁴⁰¹ steht für eine t-Alkylgruppe; R⁴⁰¹ und R⁴⁰⁶ können aneinander gebunden sein, um einen Ring zu bilden; R⁴¹², R⁴¹³, R⁴¹⁴, R⁴¹⁵ und R⁴¹⁶ stehen jeweils unabhängig für ein Wasserstoffatom oder einen Substituenten; R⁴¹¹ steht für eine t-Alkylgruppe; R⁴¹¹ und R⁴¹⁶ können aneinander gebunden sein, um einen Ring zu bilden; und R⁴²¹, R⁴²², R⁴²³ und R⁴²⁴ stehen jeweils unabhängig für ein Wasserstoffatom oder einen Substituenten, der über ein Kohlenstoffatom bindet.

13. Eine Verbindung wiedergegeben durch Formel (5): 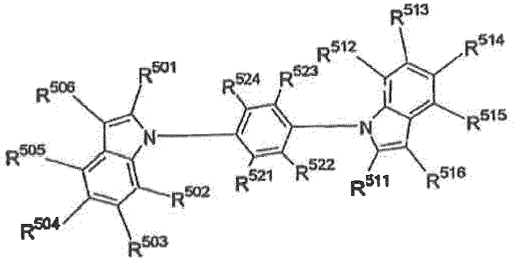 wobei R⁵⁰², R⁵⁰³, R⁵⁰⁴, R⁵⁰⁵ und R⁵⁰⁶ jeweils unabhängig für ein Wasserstoffatom oder einen Substituenten stehen; R⁵⁰¹ steht für eine t-Alkylgruppe; R⁵⁰¹ und R⁵⁰⁶ können aneinander gebunden sein, um einen Ring zu bilden; R⁵¹², R⁵¹³, R⁵¹⁴, R⁵¹⁵ und R⁵¹⁶ stehen jeweils unabhängig für ein Wasserstoffatom oder einen Substituenten; R⁵¹¹ steht für eine t-Alkylgruppe; R⁵¹¹ und R⁵¹⁶ können aneinander gebunden sein, um einen Ring zu bilden; und R⁵²¹, R⁵²², R⁵²³ und R⁵²⁴ stehen jeweils unabhängig für ein Wasserstoffatom oder einen Substituenten, der über ein Kohlenstoffatom bindet.

## Revendications

1. Un dispositif électroluminescent organique, comprenant :
une paire d'électrodes ; et
au moins une couche organique entre la paire d'électrodes, l'au moins une couche organique comprenant une couche d'émission de lumière contenant un matériau d'émission de lumière,
où l'au moins une couche organique comprend au moins une couche contenant un dérivé d'indole représenté par la formule (1) : 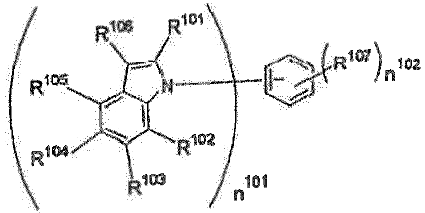 où chacun de R¹⁰², R¹⁰³, R¹⁰⁴, R¹⁰⁵ et R¹⁰⁶ représente indépendamment un atome d'hydrogène ou un substituant ; R¹⁰¹ représente un groupe alkyle ayant un atome de carbone tertiaire ou quaternaire ; R¹⁰¹ et R¹⁰⁶ peuvent être liés l'un à l'autre pour former un cycle ; R¹⁰⁷ représente un substituant ; n¹⁰¹ represente 2 ; et n¹⁰² représente un entier de 0 à 5, à la condition que n¹⁰¹ + n¹⁰² ≤ 6.

2. Le dispositif électroluminescent organique selon la revendication 1, où R¹⁰¹ représente un groupe t-butyle, un groupe isopropyle, un groupe isobutyle, un groupe isopentyle, un groupe cyclopropyle, un groupe cyclopentyle, un groupe cyclohexyle, un groupe 2-phényl-2-propyle, ou un groupe 2-(2-pyridyl)propyle.

3. Le dispositif électroluminescent organique selon la revendication 1, où R¹⁰¹ représente un groupe t-alkyle.

4. Le dispositif électroluminescent organique selon la revendication 1, où le dérivé d'indole est un composé représenté par la formule (2) : 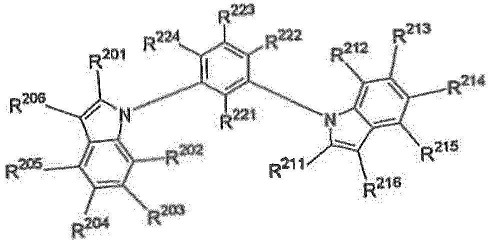 où chacun de R^{202,} R²⁰³, R²⁰⁴, R²⁰⁵ et ^{R206} représente indépendamment un atome d'hydrogène ou un substituant ; R²⁰¹ représente un groupe alkyle ayant un atome de carbone tertiaire ou quaternaire ; R²⁰¹ et R²⁰⁶ peuvent être liés l'un à l'autre pour former un cycle ; chacun de R²¹², R²¹³, R²¹⁴, R²¹⁵ et R²¹⁶ représente indépendamment un atome d'hydrogène ou un substituant ; R²¹¹ représente un groupe alkyle ayant un atome de carbone tertiaire ou quaternaire ; R²¹¹ et R²¹⁶ peuvent être liés l'un à l'autre pour former un cycle ; et chacun de R²²¹, R²²², R²²³ et R²²⁴ représente indépendamment un atome d'hydrogène ou un substituant se liant par l'intermédiaire d'un atome de carbone.

5. Le dispositif électroluminescent organique selon la revendication 4, où chacun de R²⁰¹ et R¹¹¹ représente indépendamment un groupe t-butyle, un groupe isopropyle, un groupe isobutyle, un groupe isopentyle, un groupe cyclopropyle, un groupe cyclopentyle, un groupe cyclohexyle, un groupe 2-phényl-2-propyle, ou un groupe 2-(2-pyridyl)propyle.

6. Le dispositif électroluminescent organique selon la revendication 1, où le dérivé d'indole est un composé représenté par la formule (3) : 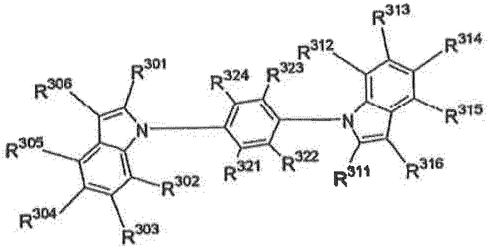 où chacun de R³⁰², R³⁰³, R³⁰⁴, R³⁰⁵ et R³⁰⁶ représente indépendamment un atome d'hydrogène ou un substituant ; R³⁰¹ représente un groupe alkyle ayant un atome de carbone tertiaire ou quaternaire ; R³⁰¹ et R³⁰⁶ peuvent être liés l'un à l'autre pour former un cycle ; chacun de R³¹², R³¹³, R³¹⁴, R³¹⁵ et R³¹⁶ représente indépendamment un atome d'hydrogène ou un substituant ; R³¹¹ représente un groupe alkyle ayant un atome de carbone tertiaire ou quaternaire ; R³¹¹ et R³¹⁶ peuvent être liés l'un à l'autre pour former un cycle ; et chacun de R³²¹, R³²², R³²³ et R³²⁴ représente indépendamment un atome d'hydrogène ou un substituant se liant par l'intermédiaire d'un atome de carbone.

7. Le dispositif électroluminescent organique selon la revendication 6, où chacun de R³⁰¹ et R³¹¹ représente indépendamment un groupe t-butyle, un groupe isopropyle, un groupe isobutyle, un groupe isopentyle, un groupe cyclopropyle, un groupe cyclopentyle, un groupe cyclohexyle, un groupe 2-phényl-2-propyle, ou un groupe 2-(2-pyridyl)propyle.

8. Le dispositif électroluminescent organique selon la revendication 1 ou 2, où le dérivé d'indole est un composé représenté par la formule (4) : 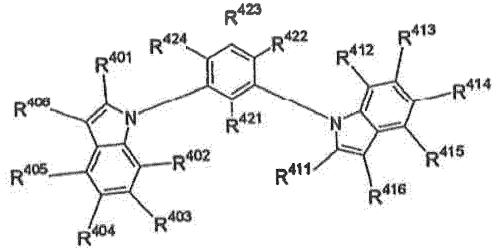 où chacun de R⁴⁰², R⁴⁰³, R⁴⁰⁴, R⁴⁰⁵ et R⁴⁰⁶ représente indépendamment un atome d'hydrogène ou un substituant ; R⁴⁰¹ représente un groupe t-alkyle ; R⁴⁰¹ et R⁴⁰⁶ peuvent être liés l'un à l'autre pour former un cycle ; chacun de R⁴¹², R⁴¹³, R⁴¹⁴, R⁴¹⁵ et R⁴¹⁶ représente indépendamment un atome d'hydrogène ou un substituant ; R⁴¹¹ représente un groupe t-alkyle ; R⁴¹¹ et R⁴¹⁶ peuvent être liés l'un à l'autre pour former un cycle ; et chacun de R⁴²¹, R⁴²², R⁴²³ et R⁴²⁴ représente indépendamment un atome d'hydrogène ou un substituant se liant par l'intermédiaire d'un atome de carbone.

9. Le dispositif électroluminescent organique selon la revendication 1 ou 2, où le dérivé d'indole est un composé représenté par la formule (5) : 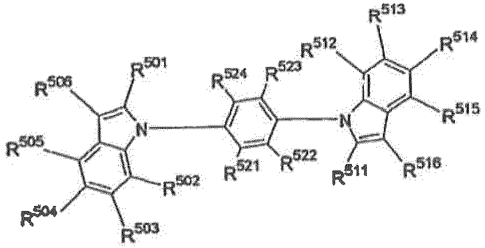 où chacun de R⁵⁰², R⁵⁰³, R⁵⁰⁴, R⁵⁰⁵ et R⁵⁰⁶ représente indépendamment un atome d'hydrogène ou un substituant ; R⁵⁰¹ représente un groupe t-alkyle ; R⁵⁰¹ et R⁵⁰⁶ peuvent être liés l'un à l'autre pour former un cycle ; chacun de R⁵¹², R⁵¹³, R⁵¹⁴, R⁵¹⁵ et R⁵¹⁶ représente indépendamment un atome d'hydrogène ou un substituant ; R⁵¹¹ représente un groupe t-alkyle ; R⁵¹¹ et R⁵¹⁶ peuvent être liés l'un à l'autre pour former un cycle ; et chacun de R⁵²¹, R⁵²², R⁵²³ et R⁵²⁴ représente indépendamment un atome d'hydrogène ou un substituant se liant par l'intermédiaire d'un atome de carbone.

10. Le dispositif électroluminescent organique selon l'une quelconque des revendications 1 à 9, où le dérivé d'indole est contenu dans la couche d'émission de lumière.

11. Le dispositif électroluminescent organique selon l'une quelconque des revendications 1 à 10, où le dérivé d'indole est contenu dans une couche contiguë à la couche d'émission de lumière.

12. Un composé représenté par la formule (4) : 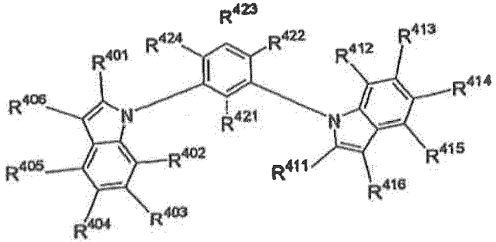 où chacun de R⁴⁰², R⁴⁰³, R⁴⁰⁴, R⁴⁰⁵ et R⁴⁰⁶ représente indépendamment un atome d'hydrogène ou un substituant ; R⁴⁰¹ représente un groupe t-alkyle ; R⁴⁰¹ et R⁴⁰⁶ peuvent être liés l'un à l'autre pour former un cycle ; chacun de R⁴¹², R⁴¹³, R⁴¹⁴, R⁴¹⁵ et R⁴¹⁶ représente indépendamment un atome d'hydrogène ou un substituant ; R⁴¹¹ représente un groupe t-alkyle ; R⁴¹¹ et R⁴¹⁶ peuvent être liés l'un à l'autre pour former un cycle ; et chacun de R⁴²¹, R⁴²², R⁴²³ et R⁴²⁴ représente indépendamment un atome d'hydrogène ou un substituant se liant par l'intermédiaire d'un atome de carbone.

13. Un composé représenté par la formule (5) : 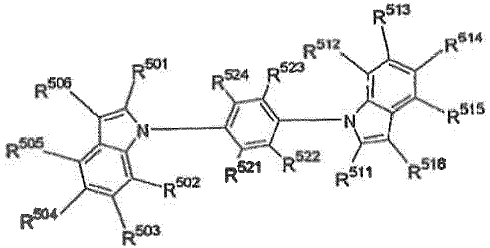 où chacun de R⁵⁰², R⁵⁰³, R⁵⁰⁴, R⁵⁰⁵ et R⁵⁰⁶ représente indépendamment un atome d'hydrogène ou un substituant ; R⁵⁰¹ représente un groupe t-alkyle ; R⁵⁰¹ et R⁵⁰⁶ peuvent être liés l'un à l'autre pour former un cycle ; chacun de R⁵¹², R⁵¹³, R⁵¹⁴, R⁵¹⁵ et R⁵¹⁶ représente indépendamment un atome d'hydrogène ou un substituant ; R⁵¹¹ représente un groupe t-alkyle ; R⁵¹¹ et R⁵¹⁶ peuvent être liés l'un à l'autre pour former un cycle ; et chacun de R⁵²¹, R⁵²², R⁵²³ et R⁵²⁴ représente indépendamment un atome d'hydrogène ou un substituant se liant par l'intermédiaire d'un atome de carbone.
